(19) Europäisches Patentamt
European Patent Office
Office européen des brevets

(11) EP 1 259 505 B1

(12) **FASCICULE DE BREVET EUROPEEN**

(45) Date de publication et mention de la délivrance du brevet: **19.05.2004 Bulletin 2004/21**

(21) Numéro de dépôt: **01907866.6**

(22) Date de dépôt: **22.02.2001**

(51) Int Cl.[7]: **C07D 401/04**, C07D 403/04, A61K 31/472, A61K 31/404, A61P 25/00, A61P 9/00, A61P 3/00

(86) Numéro de dépôt international: **PCT/FR2001/000509**

(87) Numéro de publication internationale: **WO 2001/064668 (07.09.2001 Gazette 2001/36)**

(54) **DERIVES DE 1,3-DIHYDRO-2H-INDOL-2-ONE ET LEUR UTILISATION EN TANT QUE LIGANDS DES RECEPTEURS V1B OU V1B ET V1A DE L'ARGININE-VASOPRESSINE**

1,3-DIHYDRO-2H-INDOL-2-ON DERIVATE UND DEREN VERWENDUNG ALS LIGANDEN DER ARGININ-VASOPRESSIN REZEPTOREN V1B ODER V1B UND V1A

NOVEL 1,3-DIHYDRO-2H-INDOL-2-ONE, PREPARATION METHOD AND PHARMACEUTICAL COMPOSITIONS CONTAINING SAME

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **25.02.2000 FR 0002488**

(43) Date de publication de la demande: **27.11.2002 Bulletin 2002/48**

(73) Titulaire: **SANOFI-SYNTHELABO**
**75013 Paris (FR)**

(72) Inventeurs:
- **SERRADEIL-LE GAL, Claudine**
  **F-31750 Escalquens (FR)**
- **TONNERRE, Bernard**
  **F-34570 Vailhauques (FR)**
- **WAGNON, Jean**
  **F-34000 Montpellier (FR)**

(74) Mandataire: **Thouret-Lemaitre, Elisabeth et al**
**Sanofi-Synthélabo**
**Service Brevets**
**174, avenue de France**
**75013 Paris (FR)**

(56) Documents cités:
**EP-A- 0 526 348** **WO-A-95/18105**

Remarques:
Le dossier contient des informations techniques présentées postérieurement au dépôt de la demande et ne figurant pas dans le présent fascicule.

EP 1 259 505 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention a pour objet de nouveaux dérivés de 1,3-dihydro-2*H*-indol-2-one, un procédé pour leur préparation et les compositions pharmaceutiques en contenant.

Les composés selon la présente invention présentent une affinité et une sélectivité pour les récepteurs $V_{1b}$ ou à la fois pour les récepteurs $V_{1b}$ et $V_{1a}$ de l'arginine-vasopressine (AVP).

L'AVP est une hormone connue pour son effet antidiurétique et son effet dans la régulation de la pression artérielle. Elle stimule plusieurs types de récepteurs : $V_1$ ($V_{1a}$,$V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardiovasculaires, hépatiques, pancréatiques, antidiurétiques, agrégants des plaquettes et des effets sur le système nerveux central et périphérique, et sur la sphère utérine.

La localisation des différents récepteurs est décrite dans : S. JARD et al., Vasopressin and oxytocin receptors : an overview, in Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ils ont été clonés chez le rat et l'homme et ils régulent la majorité des effets connus de l'AVP : l'agrégation plaquettaire ; les contractions utérines ; la contraction des vaisseaux ; la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire, ...).

Les récepteurs $V_{1b}$ ont été initialement identifiés dans l'adénohypophyse de différentes espèces animales (rat, porc, boeuf, mouton...) y compris chez l'homme (S. JARD et al., Mol. Pharmacol., 1986, 30, 171-177 ; Y. ARSENIJEVIC et al., J. Endocrinol., 1994, 141, 383-391 ; J. SCHWARTZ et al., Endocrinology, 1991, 129 (2), 1107-1109 ; Y. DE KEYSER et al., FEBS Letters, 1994, 356, 215-220) où ils stimulent la libération d'hormone adrénocorticotrophique par l'AVP et potentialisent les effets du CRF sur la libération d'ACTH (G.E. GILLIES et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. DE KEYSER, FEBS Letters, 1994, 356, 215-220 ; T. SUGIMOTO et al., J. Biol. Chem., 1994, 269 (43), 27088-27092 ; M. SAITO et al., Biochem. Biophys. Res. Commun., 1995, 212 (3), 751-757 ; S.J. LOLAIT et al., Neurobiology, 1996, 92, 6783-6787 ; M.A. VENTURA et al., Journal of Molecular endocrinology, 1999, 22, 251-260) et différentes études (hybridation *in situ,* PCR, de l'anglais Polymerase Chain Reaction, ...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régulait le pancréas endocrine en stimulant la sécrétion d'insuline et de glucagon (B. LEE et al., Am. J. Physiol. 269 (Endocrinol. Metab. 32) : E1095-E1100, 1995) ou la production de catécholamines dans la medullo-surrénale qui est le siège d'une synthèse locale d'AVP (E. GRAZZINI et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétants de l'AVP et induisant de ce fait une production soutenue de catécholamines à l'origine d'hypertensions résistantes aux antagonistes des récepteurs de l'angiotensine II et aux inhibiteurs de l'enzyme de conversion. Le cortex surrénalien est aussi riche en récepteurs $V_{1a}$ impliqués dans la production de gluco- et minéralo-corticoides (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1b}$ et/ou $V_{1a}$ portés par les cellules de la médulla (G. MAZZOCCHI et al., Peptides, 1997, 18 (2), 191-195 ; E. GRAZZINI et al., J. Clin. Endocrinol. Metab., 1999, 84 (6), 2195-2203).

Les récepteurs $V_{1b}$ sont aussi considérés comme un marqueur des tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroidiens, induisant un syndrome de Cushing dans certains cas (J. BERTHERAT et al., Eur. J. Endocrinol., 1996, 135,173 ; G.A. WITTERT et al., Lancet, 1990, 335, 991-994 ; G. DICKSTEIN et al., J. Clin. Endocrinol. Metab., 1996, 81 (8), 2934-2941). Les récepteurs $V_{1a}$ sont, quant à eux, un marqueur plus spécifique des cancers pulmonaires à petites cellules (SCLC) (P.J. WOLL et al., Biochem.

Biophys. Res. Commun., 1989, 164 (1), 66-73). Ainsi, les composés selon la présente invention sont des outils de diagnostic évidents et offrent une approche thérapeutique nouvelle dans la prolifération et la détection de ces tumeurs, à un stade même précoce (radiomarquage ; SPECT, de l'anglais Single Photon Emission Computed Tomography ; PET Scan, de l'anglais Positron Emission Tomography Scanner).

La présence abondante du messager des récepteurs $V_{1b}$ au niveau stomacal et intestinal, suggère une implication de l'AVP via ce récepteur sur la libération d'hormones gastrointestinales comme la cholécystokinine, la gastrine ou la sécrétine (T. SUGIMOTO et al., Molecular cloning and functional expression of $V_{1b}$ receptor gene, dans Neurohypophysis : Recent Progress of Vasopressin and Oxytocin Research ; T. SAITO, K. KUROKAWA and S. YOSHIDA ed., Elvesier Science, 1995, 409-413).

Des dérivés de 1,3-dihydro-2*H*-indol-2-one ont été décrits dans certaines demandes de brevet comme des ligands des récepteurs de l'arginine-vasopressine et/ou de l'ocytocine : on peut citer les demandes de brevets WO 93/15051, EP 636608, EP 636609, WO 95/18105, WO 97/15556 et WO 98/25901.

A ce jour, aucun composé non peptidique ayant une affinité et une sélectivité pour les récepteurs $V_{1b}$ ou à la fois pour les récepteurs $V_{1b}$ et $V_{1a}$ de l'arginine-vasopressine n'est connu.

On a maintenant trouvé de nouveaux dérivés de 1,3-dihydro-2*H*-indol-2-one qui présentent une affinité et une sélectivité pour les récepteurs $V_{1b}$ ou à la fois pour les récepteurs $V_{1b}$ et $V_{1a}$ de l'arginine-vasopressine.

Ces composés peuvent être utilisés pour la préparation de médicaments utiles dans le traitement ou la prévention de toute pathologie où l'arginine-vasopressine et/ou les récepteurs $V_{1b}$ ou à la fois les récepteurs $V_{1b}$ et les récepteurs $V_{1a}$ sont impliqués, notamment dans le traitement ou la prévention des affections du système cardiovasculaire, par exemple l'hypertension ; du système nerveux central par exemple, le stress, l'anxiété, la dépression, le trouble obsessionnel-compulsif, les attaques de panique ; du système rénal ; du système gastrique ainsi que dans le traitement des cancers pulmonaires à petites cellules ; de l'obésité ; du diabète de type II ; de la résistance à l'insuline ; de l'hypertriglycéridémie ; de l'athérosclérose ; du syndrome de Cushing ; de toutes pathologies consécutives au stress et des états de stress chroniques.

Ainsi, selon un de ses aspects, la présente invention a pour objet des composés de formule (Ia) sous forme d'isomère lévogyre :

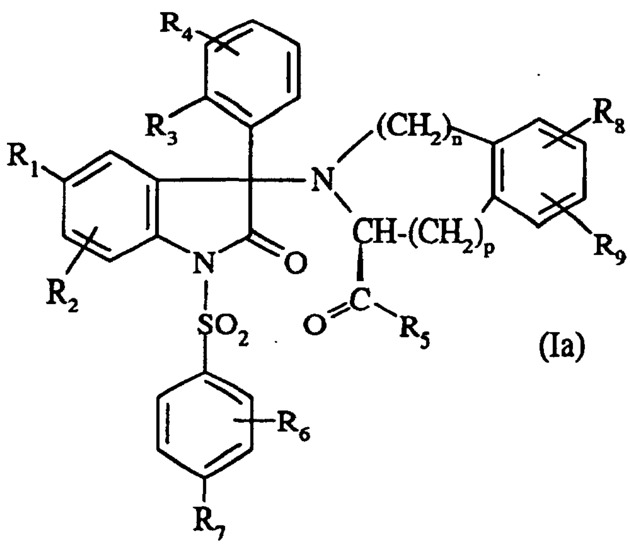

(Ia)

dans laquelle :
l'atome de carbone portant le substituant $COR_5$ a la configuration (S) ;

- le radical :

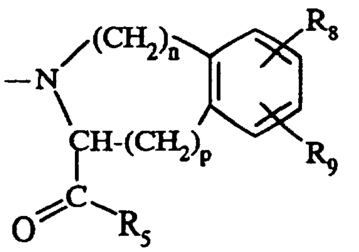

représente l'un des radicaux suivants :

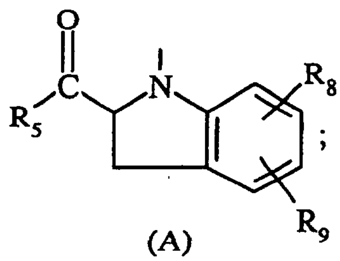

(A)

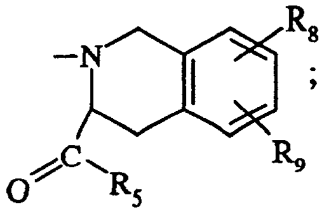

;

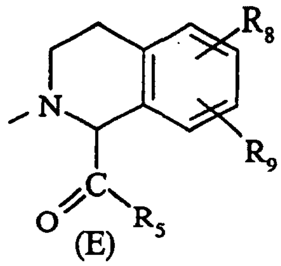

(D) (E)

- $R_1$ représente un atome de chlore ou un radical méthyle ;
- $R_2$ représente un atome d'hydrogène ou est en position -4- ou -6- de l'indol-2-one et représente un atome de chlore, un radical méthyle ou un radical méthoxy ;
- $R_3$ représente un radical méthoxy ou un atome de chlore ;
- $R_4$ représente un atome d'hydrogène ou est en position -3- ou -4- du phényle et représente un radical méthoxy ;
- ou bien $R_4$ est en position -3- du phényle et ensemble avec $R_3$ représentent un radical méthylènedioxy ;
- $R_5$ représente un groupe diméthylamino ou un radical méthoxy ;
- $R_6$ est en position -2- du phényle et représente un radical méthoxy ;
- $R_7$ représente un radical méthoxy ;
- $R_8$ et $R_9$ représentent un atome d'hydrogène ;

ainsi que leurs solvats et/ou hydrates.

Les composés suivants :

- (3S)-2-[5-Chloro-1-[(2,4-diméthoxyphényl)sulfonyl]-3-(2-méthoxyphényl)-2-oxo-2,3-dihydro-1*H*-indol-3-yl]-N,N-diméthyl-1,2,3,4-tétrahydroisoquinoléine-3-carboxamide, isomère lévogyre ;
- (1S)-2-[5-Chloro-1-[(2,4-diméthoxyphényl)sulfonyl)-3-(2-méthoxyphényl)-2-oxo-2,3-dihydro-1*H*-indol-3-yl]-N,N-diméthyl-1,2,3,4-tétrahydroisoquinoléine-1-carboxamide, isomère lévogyre ;
- (2S)-1-[5-Chloro-1-[(2,4-diméthoxyphényl)sulfonyl]-3-(2-méthoxyphényl)-2-oxo-2,3-dihydro-1*H*-indol-3-yl]-N,N-diméthyl-2,3-dihydro-1H-indole-2-carboxamide, isomère lévogyre ;
- Ester méthylique de l'acide (3S)-2-[5-chloro-1-[(2,4-diméthoxyphényl)sulfonyl]-3-(2-méthoxyphényl)-2-oxo-2,3-dihydro-1*H*-indol-3-yl]-1,2,3,4-tétrahydroiso quinoléine-3-carboxylique, isomère lévogyre ;
- (3S)-2-[5,6-Dichloro-1-[(2,4-diméthoxyphényl)sulfonyl]-3-(2-méthoxyphényl)-2-oxo-2,3-dihydro-1*H*-indol-3-yl]-N,N-diméthyl-1,2,3,4-tétrahydroisoquinoléine-3-carboxamide, isomère lévogyre ;

- (3S)-2-[5-Chloro-1-[(2,4-diméthoxyphényl)sulfonyl]-3-(2-méthoxyphényl)-6-méthyl-2-oxo-2,3-dihydro-1*H*-indol-3-yl]-N,N-diméthyl-1,2,3,4-tétrahydro isoquinoléine-3-carboxamide, isomère lévogyre ;
- (3S)-2-[5-Chloro-3-(2-chlorophényl)-1-[(2,4-diméthoxyphényl)sulfonyl]-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-1-[(2,4-diméthoxyphényl)sulfonyl]-3-(2,3-diméthoxyphényl)-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-1-[(2,4-diméthoxyphényl)sulfonyl]-3-(2,4-diméthoxyphényl)-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-3-(1,3-benzodioxol-4-yl)-1-[(2,4-diméthoxyphényl)sulfonyl]-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5,6-Dichloro-3-(2-chlorophényl)-1-[(2,4-diméthoxyphényl)sulfonyl]-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-3-(2-chlorophényl)-1-[(2,4-diméthoxyphényl)sulfonyl]-6-méthoxy-2-oxo-2,3-dihydro-1*H*-indol-3-yl]-N,N-diméthyl-1,2,3,4-tétrahydroiso quinoléine-3-carboxamide, isomère lévogyre ;
- (3S)-2-[4-Chloro-1-[(2,4-diméthoxyphényl)sulfonyl]-3-(2-méthoxyphényl)-5-méthyl-2-oxo-2,3-dihydro-1*H*-indol-3-yl]-N,N-diméthyl-1,2,3,4-tétrahydroiso quinoléine-3-carboxamide, isomère lévogyre ;

ainsi que leurs solvats et/ou hydrates sont plus particulièrement préférés.

Selon un autre de ses aspects, la présente invention a pour objet un procédé de préparation des composés de formule (Ia) isomère lévogyre, de leurs solvats et/ou leurs hydrates caractérisé en ce que :

on fait réagir, en présence d'une base, un composé de formule :

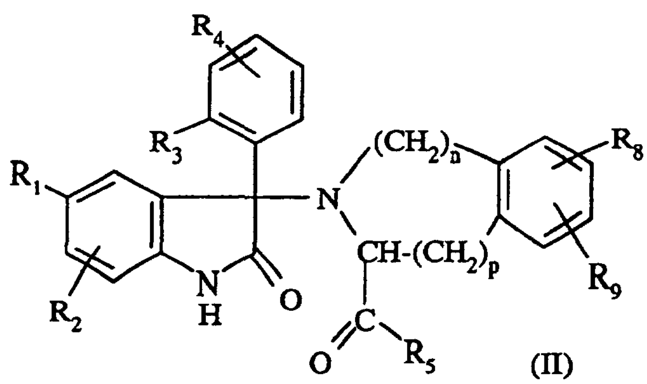

(II)

dans laquelle l'atome de carbone portant le substituant -$COR_5$ a la configuration (S), n, p, $R_1$, $R_2$, $R_3$, $R_4$, $R_5$, $R_8$ et $R_9$ sont tels que définis pour un composé de formule (Ia) isomère lévogyre, avec un halogénure de formule :

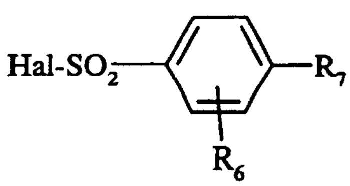

(III)

dans laquelle $R_6$ et $R_7$ sont tels que définis pour un composé de formule (Ia) isomère lévogyre et Hal représente un atome d'halogène.

La réaction s'effectue en présence d'une base forte comme un hydrure métallique tel que l'hydrure de sodium ou un alcoolate alcalin tel que le *tert*-butylate de potassium, dans un solvant anhydre tel que le N,N-diméthylformamide ou le tétrahydrofurane et à une température comprise entre -70°C et +60°C. La réaction s'effectue de préférence en utilisant un composé de formule (III) dans laquelle Hal = Cl.

Les composés de formule (Ia) isomère lévogyre ainsi obtenus peuvent être ultérieurement séparés du milieu réactionnel et purifiés selon les méthodes classiques, par exemple par cristallisation ou chromatographie.

Les composés de formule (II) se préparent par réaction d'un composé 3-halogéno-1,3-dihydro-2*H*-indol-2-one de formule :

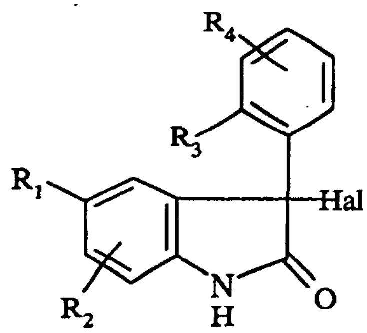

(IV)

dans laquelle $R_1$, $R_2$, $R_3$ et $R_4$ sont tels que définis pour un composé de formule (Ia) isomère lévogyre et Hal représente un atome d'halogène, de préférence le chlore ou le brome, avec un composé de formule :

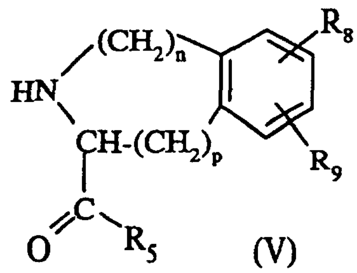

(V)

dans laquelle l'atome de carbone portant le substituant $-COR_5$ a la configuration (S), n, p et $R_5$, $R_8$ et $R_9$ sont tels que définis pour un composé de formule (Ia). La réaction s'effectue en présence d'une base telle que la diisopropyléthylamine ou la triéthylamine, dans un solvant inerte tel que le dichlorométhane, le chloroforme ou le tétrahydrofurane ou un mélange de ces solvants et à une température comprise entre la température ambiante et la température de reflux du solvant.

Les composés de formule (III) sont connus ou préparés par des méthodes connues telles que celles décrites dans EP-0 469 984 B et WO 95/18105. Par exemple, les composés de formule (III) peuvent être préparés par halogénation des acides benzènesulfoniques correspondants ou de leurs sels, par exemple de leurs sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant inerte tel qu'un hydrocarbure halogéné ou le N,N-diméthylformamide et à une température comprise entre -10°C et 200°C.

Le chlorure de 2,4-diméthoxybenzènesulfonyle est préparé selon J. Am. Chem. Soc., 1952, 74, 2008.

Les composés de formule (IV) sont connus et se préparent selon des méthodes connues telles que celles décrites dans WO 95/18105.

Par exemple, on transforme un composé de formule :

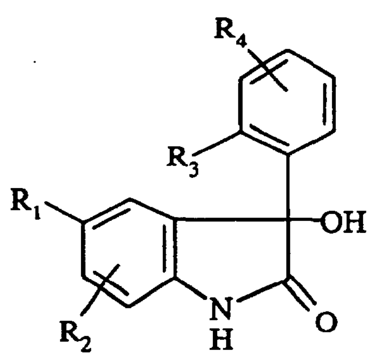

(VI)

dans laquelle $R_1$, $R_2$, $R_3$ et $R_4$ sont tels que définis pour un composé de formule (Ia) isomère lévogyre, en un composé

de formule (IV) dans laquelle Hal = Cl par action du chlorure de thionyle en présence d'une base telle que la pyridine, dans un solvant inerte tel que le dichlorométhane et à une température comprise entre 0°C et la température ambiante.

Selon un autre exemple de préparation des composés de formule (IV), on transforme un comnosé de formule :

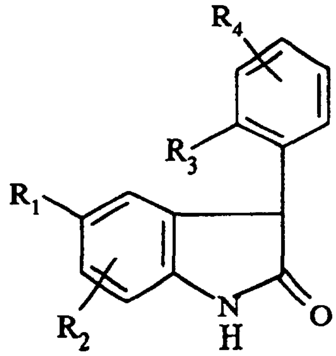

(VII)

dans laquelle $R_1$, $R_2$, $R_3$ et $R_4$ sont tels que définis pour un composé de formule (Ia) isomère lévogyre, en un composé de formule (IV) au moyen d'un agent d'halogénation comme le brome selon le procédé décrit dans Farm. Zh.(Kiev), 1976, 5, 30-33.

Les composés de formule (VI) sont connus et se préparent selon des méthodes connues telles que celles décrites dans WO 95/18105.

Par exemple, on prépare un composé de formule (VI) par réaction d'un dérivé de 1*H*-indole-2,3-dione de formule :

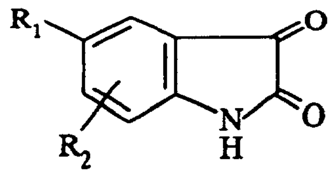

(VIII)

dans laquelle $R_1$ et $R_2$ sont tels que définis pour un composé de formule (Ia) isomère lévogyre, avec un dérivé organomagnésien de formule :

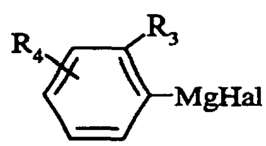

(IX)

dans laquelle $R_3$ et $R_4$ sont tels que définis pour un composé de formule (Ia) isomère lévogyre et Hal représente un atome d'halogène, de préférence le brome ou l'iode, dans un solvant inerte tel que le tétrahydrofurane ou l'éther diéthylique et à une température comprise entre 0°C et la température de reflux du solvant.

On peut également préparer un composé de formule (VI) dans laquelle $R_3$ est tel que défini pour un composé de formule (Ia) isomère lévogyre et $R_4$, différent de l'hydrogène, est en position -3- du phényle, par réaction d'un composé de formule :

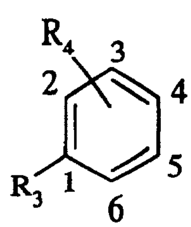

(XVI)

dans laquelle $R_3$ est tel que défini pour un composé de formule (Ia) isomère lévogyre et $R_4$ est en position -2- du phényle, avec un dérivé du lithium tel que le n-butyllithium, puis l'intermédiaire lithié ainsi obtenu est mis en réaction

avec un composé de formule (VIII). La réaction s'effectue dans un solvant tel que l'éther diéthylique, le tétrahydrofurane, l'hexane ou un mélange de ces solvants, à une température comprise entre -70°C et la température ambiante.

Les dérivés de 1*H*-indole-2,3-dione (VIII) sont commerciaux ou se préparent selon les méthodes décrites dans Tetrahedron Letters, 1998, 39, 7679-7682 ; Tetrahedron Letters, 1994, 35, 7303-7306 ; J. Org. Chem., 1977, 42 (8), 1344-1348 ; J. Org. Chem., 1952, 17, 149-156 ; J. Am. Chem. Soc., 1946, 68, 2697-2703, Organic Syntheses, 1925, V, 71-74 et Advances in Heterocyclic Chemistry, A.R. Katritzky and A.J. Boulton, Academic Press, New-York, 1975, 18, 2-58.

Les dérivés organomagnésiens (IX) sont préparés selon les méthodes classiques bien connues de l'homme de l'art.

On peut également préparer un composé de formule (VI), par oxydation par l'air d'un composé de formule (VII) en présence d'une base telle que l'hydrure de sodium et en présence de diméthyldisulfure.

De façon particulière, on peut préparer les composés de formule (VI) dans laquelle $R_3$ = méthoxy et $R_4$ = H, ou bien $R_3$ = $R_4$ = méthoxy avec $R_4$ en position -3 du phényle, $R_2$ est différent d'un atome de chlore et $R_1$ est tel que défini pour un composé de formule (Ia) isomère lévogyre, en suivant le procédé décrit dans le SCHEMA 1.

SCHEMA 1

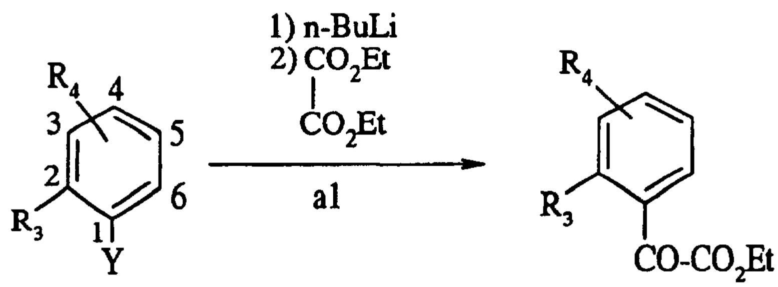


(X) : $R_3$ = méthoxy, $R_4$ = H ;
$R_3$ = $R_4$ = méthoxy avec
$R_4$ en position -3 ;
Y = H ou Br.

(XI)

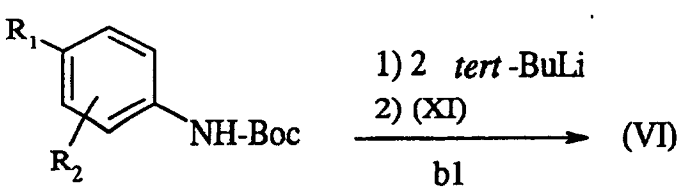


(XII)

A l'étape a1 du SCHEMA 1, on fait d'abord réagir un composé de formule (X) avec un dérivé du lithium tel que le n-butyllithium, en l'absence ou en présence d'une base telle que le N,N,N'N'-tétraméthylènediamine, puis l'intermédiaire lithié ainsi obtenu est mis en réaction avec l'oxalate de diéthyle pour donner le composé de formule (XI). La réaction s'effectue dans un solvant inerte tel que l'éther diéthylique ou le tétrahydrofurane et à une température comprise entre -70°C et la température ambiante.

A l'étape b1, on fait d'abord réagir un composé de formule (XII) avec deux équivalents d'un dérivé du lithium tel que le *tert*-butyllithium, puis l'intermédiaire lithié ainsi obtenu est mis en réaction avec le composé de formule (XI) pour donner le composé de formule (VI) attendu. La réaction s'effectue dans un solvant inerte tel que l'éther diéthylique ou le tétrahydrofurane et à une température comprise entre -70°C et la température ambiante.

Les composés dé formule (X) sont commerciaux ou synthétisés de manière classique.

Les composés de formule (XII) sont préparés par réaction des dérivés de l'aniline correspondants avec du di-*tert*-butyldicarbonate selon les méthodes classiques.

Les composés de formule (VII) sont connus et se préparent selon des méthodes connues telles que celles décrites dans WO 95/18105 ou dans J. Org. Chem., 1968, 33, 1640-1643.

Les composés de formule (V) sont connus ou se préparent selon des méthodes connues. Ainsi, par exemple, les composés de formule (V) dans laquelle $R_5$ représente un groupe diméthylamino se préparent selon le SCHEMA 2 ci-après dans lequel Pr représente un groupe N-protecteur, en particulier le *tert*-butoxycarbonyle ou le 9-fluorénylméthoxycarbonyle et n, p, $R_8$ et $R_9$ sont tels que définis pour un composé de formule (Ia) isomère lévogyre.

SCHEMA 2

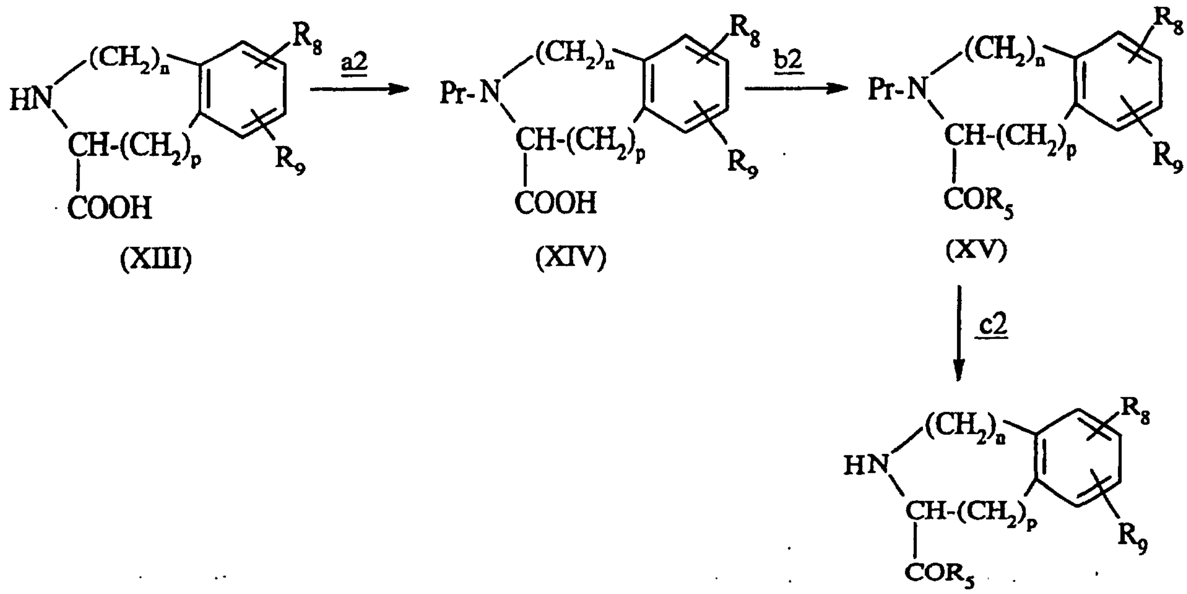


(XIII) (XIV) (XV)

(V)

A l'étape a2 du SCHEMA 2, on protège l'atome d'azote du composé de formule (XIII) selon les méthodes classiques pour obtenir un composé de formule (XIV). Parmi les composés de formule (XIV), certains sont commerciaux.

L'acide (XIV) est mis en réaction à l'étape b2 avec la diméthylamine selon les méthodes classiques du couplage peptidique pour donner le composé de formule (XV), qui est déprotégé à l'étape c2, selon les méthodes connues, pour donner le composé de formule (V) attendu. En particulier, lorsque Pr représente un groupe 9-fluorénylméthoxycarbonyle, on effectue la déprotection en utilisant la méthode décrite dans Synthetic Communications, 1994, 24 (2), 187-195.

Les composés de formule (V) dans laquelle $R_5$ représente un méthoxy sont connus ou se préparent selon des méthodes connues comme, par exemple, par réaction d'estérification à partir des acides de formule (XIII) ou selon les méthodes décrites dans Tetrahedron Letters, 1986, 27, 2409-2410 ; J. Am. Chem. Soc., 1970, 92, 2476-2488 ; Tetrahedron : Asymmetry, 1998, 9, 4295-4299 ; J. Med. Chem., 1994, 37, 3956-3968 ; J. Chem. Soc., Perkin Trans 1, 1977, 596-600 ; Gazz. Chim. Ital., 1976, 106, 65 ; Chem. Pharm. Bull., 1983, 31, 312-314 ; J. Med. Chem., 1983, 26, 1267-1277 ; J. Org. Chem., 1997, 62, 7679-7689 ; J. Med. Chem., 1992, 35, 1942-1953 ; Justus Liebigs Ann. Chem., 1976, 367-382 ; J. Org. Chem., 1978, 43, 2115-2119 ; Tetrahedron Letters, 1997, 38, 6977-6980 ; Helv. Chim. Acta, 1959, 42, 2431-2436.

Les acides de formule (XIII) sont commerciaux ou se préparent selon des méthodes connues. Ainsi, par exemple :

- Les acides 2,3-dihydro-1*H*-indole-2-carboxylique se préparent selon J. Med. Chem., 1983, 26, 394-403 ; Agric. Biol. Chem., 1987, 51, 1833-1838 ; J. Med. Chem., 1983, 26, 1267-1277 ; Helv. Chim. Acta, 1962, 45, 638 ; Helv. Chim. Acta, 1968, 51, 1476 ;
- Les acides 1,2,3,4-tétrahydroisoquinoléine-3-carboxylique se préparent selon Synthesis, 1992, 11, 1157-1160 ; Int. J. Peptide Protein Res., 1994, 43, 62-68 ; Liebigs Ann./Recueil, 1997, 3, 533-540 ; J. Med. Chem., 1988, 31, 2092-2097 ; J. Chem. Soc., 1938, 172-175 ; J. Chem. Soc., 1950, 1534-1537 ; Synthesis, 1990, 550-556 ; Heterocycles, 1992, 34, 757-764 ; J. Med. Chem., 1983, 26, 1267-1277 ;
- Les acides 1,2,3,4-tétrahydroisoquinoléine-1-carboxylique se préparent selon J. Med. Chem., 1993, 36, 314-319

ou selon WO 93/12091.

Lorsque l'on souhaite préparer un composé de formule (Ia) optiquement pur, on fait réagir, de préférence, un composé de formule (II) optiquement pur avec un composé de formule (III) selon le procédé de l'invention.

Les composés de formule (II) optiquement purs se préparent par réaction du composé de formule (IV) racémique avec un composé de formule (V) optiquement pur, puis séparation du mélange des diastéréoisomères selon les méthodes classiques, par exemple par cristallisation ou chromatographie.

Alternativement, on peut faire réagir le mélange des diastéréoisomères du composé de formule (II) avec le composé de formule (III) et séparer le mélange des diastéréoisomères du composé de formule (Ia) ainsi obtenu.

Au cours de l'une quelconque des étapes de préparation des composés de formule (Ia) isomère lévogyre ou des composés intermédiaires de formule (II), (IV), (V) ou (VI), il peut être nécessaire et/ou souhaitable de protéger les groupes fonctionnels réactifs ou sensibles, tels que les groupes amine, hydroxyle ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973, dans Protective Groups in Organic Synthesis, T.W. Greene et P.G.M. Wutts, Ed. John Wiley et sons, 1991 ou dans Protecting Groups, Kocienski P.J., 1994, Georg Thieme Verlag. L'élimination des groupes protecteurs peut s'effectuer à une étape ultérieure opportune en utilisant les méthodes connues de l'homme de l'art et qui n'affectent pas le reste de la molécule concernée.

Les groupes N-protecteurs éventuellement utilisés sont les groupes N-protecteurs classiques bien connus de l'homme de l'art tels que par exemple le groupe *tert*-butoxycarbonyle, fluorénylméthoxycarbonyle, benzyle, benzhydrylidène ou benzyloxycarbonyle.

Les composés de formule (II) sont nouveaux et font partie de l'invention.

Ainsi, selon un autre de ses aspects, l'invention a pour objet des composés de formule :

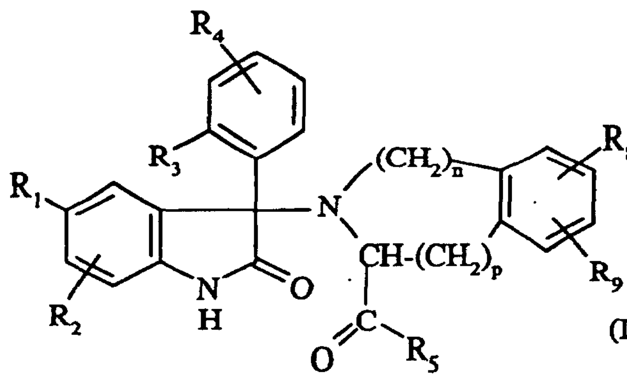

(II)

dans laquelle :

- l'atome de carbone portant le substituant $-COR_5$ a la configuration (S) ;
- le radical

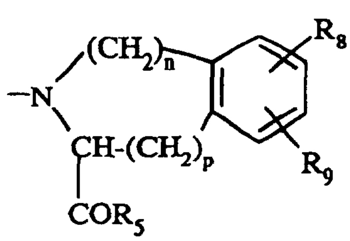

,

$R_1$, $R_2$, $R_3$, $R_4$, $R_5$, $R_8$ et $R_9$ sont tels que définis pour un composé de formule (Ia) isomère lévogyre ;

ainsi que leurs sels avec des acides minéraux ou organiques, sous forme d'isomères optiquement purs ou sous forme de mélange de diastéréoisomères ou sous forme de mélange racémique.

Les sels des composés de formule (II) comprennent ceux avec des acides minéraux ou organiques qui permettent une séparation ou une cristallisation convenable des composés de formule (II) tels que le chlorhydrate, le bromhydrate, l'oxalate, le maléate, le succinate, le fumarate, le citrate, l'acétate.

Les composés de formule (Ia) isomère lévogyre ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'études biochimiques.

L'affinité des composés de formule (Ia) isomère lévogyre selon l'invention pour les récepteurs $V_{1b}$ de l'arginine-vasopressine a été déterminée *in vitro* en utilisant la méthode décrite par Y. DE KEYSER et al., Febs Letters, 1994, 356, 215-220. Cette méthode consiste à étudier *in vitro* le déplacement de l'arginine-vasopressine tritiée ([$^3$H]-AVP) aux récepteurs $V_{1b}$ présents sur des préparations membranaires adénohypophysaires ou cellulaires portant les récepteurs $V_{1b}$ de rat ou humains. Les concentrations inhibitrices de 50 % ($CI_{50}$) de la fixation de l'arginine-vasopressine tritiée des composés selon l'invention sont faibles et varient de $10^{-6}$ à $10^{-9}$M plus particulièrement de $10^{-7}$ à $10^{-9}$M.

L'affinité des composés de formule (Ia) isomère lévogyre selon l'invention pour les récepteurs $V_{1a}$ de l'arginine-vasopressine a été déterminée *in vitro* en utilisant la méthode décrite par M. THIBONNIER et al., J. Biol. Chem., 1994, 269, 3304-3310. Cette méthode consiste à étudier *in vitro* le déplacement de l'arginine-vasopressine tritiée ([$^3$H]-AVP) aux récepteurs $V_{1a}$ présents sur des préparations membranaires ou cellulaires portant les récepteurs $V_{1a}$ de rat ou humains. Parmi les composé de formule (Ia) isomère lévogyre, certains présentent aussi une affinité pour les récepteurs $V_{1a}$ de l'arginine-vasopressine avec des $CI_{50}$ qui varient de $10^{-6}$ à $10^{-9}$ M plus particulièrement de $10^{-7}$ à $10^{-8}$M.

L'affinité des composés de formule (Ia) isomère lévogyre selon l'invention pour les récepteurs $V_2$ de la vasopressine a également été étudiée (méthode décrite par M. Birnbaumer et al., Nature (Lond.), 1992, 357, 333-335). Les composés étudiés sont peu ou pas affins pour les récepteurs $V_2$.

Les composés de la présente invention sont notamment des principes actifs de compositions pharmaceutiques, dont la toxicité est compatible avec leur utilisation en tant que médicaments.

Selon un autre de ses aspects, la présente invention concerne l'utilisation des composés de formule (Ia) isomère lévogyre, de leurs solvats et/ou hydrates pharmaceutiquement acceptables, pour la préparation de médicaments destinés à traiter toute pathologie où l'arginine-vasopressine et/ou ses récepteurs $V_{1b}$ ou à la fois ses récepteurs $V_{1b}$ et ses récepteurs $V_{1a}$ sont impliqués.

Selon un autre de ses aspects, la présente invention concerne l'utilisation des composés de formule (Ia) isomère lévogyre, de leurs solvats et/ou hydrates pharmaceutiquement acceptables pour la préparation de médicaments destinés à traiter les pathologies du système cardiovasculaire, du système nerveux central, du système rénal, du système gastrique ainsi que les cancers du poumon à petites cellules, l'obésité, le diabète de type II, la résistance à l'insuline, l'hypertriglycéridémie, l'athérosclérose, le syndrome de Cushing, toutes pathologies consécutives au stress et les états de stress chroniques.

Ainsi les composés selon l'invention peuvent être utilisés, chez l'homme ou chez l'animal, dans le traitement ou la prévention de différentes affections vasopressine-dépendantes tels que les affections cardiovasculaires, comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase ; les affections du système nerveux central comme la migraine, le vasospasme cérébral, l'hémorragie cérébrale, les oedèmes cérébraux, la dépression, l'anxiété, le stress, le trouble obsessionnel-compulsif, les attaques de panique, les états psychotiques, les troubles de la mémoire par exemple ; les affections du système rénal comme le vasospasme rénal, la nécrose du cortex rénal, le diabète insipide néphrogénique ; les affections du système gastrique comme le vasospasme gastrique, l'hépatocirrhose, les ulcères, la pathologie des vomissements, par exemple la nausée y compris la nausée due à une chimiothérapie, le mal des transports ; la néphropathie diabétique. Les composés'selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules ; des encéphalopathies hyponatrémiques ; du syndrome pulmonaire, de la maladie de Ménière ; du glaucome, de la cataracte ; de l'obésité ; du diabète de type II ; de l'athérosclérose ; du syndrome de Cushing ; de la résistance à l'insuline ; de l'hypertriglycéridémie ; dans les traitements post-opératoires, notamment après une chirurgie abdominale.

Les composés selon l'invention peuvent aussi être utilisés dans le traitement ou la prévention de toutes les pathologies consécutives au stress comme la fatigue et ses syndrômes, les désordres ACTH dépendant, les troubles cardiaques, la douleur, les modifications de la vidange gastrique, de l'excrétion fécale (colite, syndrôme du colon irritable, maladie de Crohn), de la sécrétion acide, l'hyperglycémie, l'immunosuppression, les processus inflammatoires (arthrite rhumatoïde et ostéoarthrite), les infections multiples, les cancers, l'asthme, le psoriasis, les allergies et les désordres neuropsychiatriques variés tel que l'anorexie nerveuse, la boulimie, les troubles de l'humeur, la dépression,

l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysosurrénalien.

Les composés selon l'invention peuvent également être utilisés comme psychostimulants, provoquant l'augmentation de l'éveil, la réactivité émotionnelle face à l'environnement et facilitant l'adaptation.

Les composés de formule (Ia) isomère lévogyre ci-dessus, leurs solvats et/ou hydrates pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (Ia) isomère lévogyre sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (Ia) isomère lévogyre, un de ses solvats et/ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose, la cellulose microcristalline, l'amidon, le phosphate dicalcique, de liants comme par exemple la polyvinylpyrrolidone, l'hydroxypropylméthylcellulose, des délitant comme la polyvinylpyrrolidone réticulée, la carboxyméthylcellulose réticulée, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un

tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lequel le principe actif peuvent être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, $\alpha$, $\beta$, $\gamma$-cyclodextrine, 2-hydroxypropyl-$\beta$-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (Ia) isomère lévogyre est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Les compositions de la présente invention peuvent contenir, à côté des composés de formule (Ia) isomère lévogyre, de leurs solvats et/ou hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur les récepteurs du CRF.

Les composés selon l'invention pourront également être utilisés pour la préparation de compositions à usage vétérinaire.

Les PREPARATIONS et EXEMPLES suivants illustrent l'invention sans toute fois la limiter.

Dans les Préparations et dans les Exemples on utilise les abréviations suivantes :

éther : éther diéthylique
éther iso : éther diisopropylique
DMF : N,N-diméthylformamide
THF : tétrahydrofurane
DCM : dichlorométhane
AcOEt : acétate d'éthyle
DIPEA : diisopropyléthylamine
TFA : acide trifluoroacétique
Boc : *tert*-butoxycarbonyle
Cbz : benzyloxycarbonyle
BOP : benzotriazol-1-yloxytris(diméthylamino)phosphonium hexafluorophosphate
PyBOP : benzotriazol-1-yloxytripyrrolidinophosphonium hexafluorophosphate
DCC : 1,3-dicyclohexylcarbodiimide
HOBT : 1-hydroxybenzotriazole hydrate
F : point de fusion
TA : température ambiante
Eb : température d'ébullition
CLHP : chromatographie liquide haute performance.

Les spectres de résonance magnétique du proton (RMN $^1H$) sont enregistrés à 200 MHz dans du DMSO-$d_6$, en utilisant le pic du DMSO-$d_6$ comme référence. Les déplacements chimiques $\delta$ sont exprimés en parties par million

(ppm). Les signaux observés sont exprimés ainsi : s: singulet ; se : singulet élargi ; d : doublet ; d.d : doublet dédoublé ; t : triplet ; q : quadruplet ; m : massif ; mt : multiplet.

PREPARATIONS

Préparations des composés de formule (IV).

Préparation 1.1

3,5-Dichloro-3-(2-méthoxyphényl)-1,3-dihydro-2*H*-indol-2-one.

(IV) : $R_1$ = Cl ; $R_2$ = H ; $R_3$ = $OCH_3$ ; $R_4$ = H ; Hal = Cl.

A) 5-Chloro-3-hydroxy-3-(2-méthoxyphényl)-1,3-dihydro-2*H*-indol-2-one.

On prépare ce composé selon le mode opératoire décrit dans WO 95/18105. On prépare une solution de bromure de 2-méthoxyphénylmagnésium à partir de 16 g de magnésium dans 35 ml d'éther et d'une solution de 124 g de 1-bromo-2-méthoxybenzène dans 175 ml d'éther. On ajoute, en goutte à goutte, sous atmosphère d'argon, cette solution à un mélange de 30 g de 5-chloro-1*H*-indole-2,3-dione dans 250 ml de THF, préalablement refroidi au bain de glace, puis laisse sous agitation en laissant remonter la température à TA. Après 1 heure d'agitation à TA, on verse lentement le mélange réactionnel sur une solution saturée de $NH_4Cl$ et évapore le THF sous vide. On essore le précipité formé et le lave à l'éther iso. On obtient 42 g du produit attendu que l'on utilise tel quel à l'étape suivante.

B) 3,5-Dichloro-3-(2-méthoxyphényl)-1,3-dihydro-2*H*-indol-2-one.

On prépare ce composé selon le mode opératoire décrit dans WO 95/18105. On refroidit à 0°C un mélange de 12,71 g du composé obtenu à l'étape précédente dans 105 ml de DCM, ajoute 5,3 ml de pyridine puis, 4,9 ml de chlorure de thionyle. Après 30 minutes sous agitation, on ajoute de l'eau au mélange réactionnel et évapore le DCM sous vide. On essore le précipité formé, le lave trois fois à l'eau puis trois fois à l'éther iso et le sèche. On obtient 13,66 g du produit attendu que l'on utilise tel quel.

Préparation 1.2

3,5,6-Trichloro-3-(2-méthoxyphényl)-1,3-dihydro-2*H*-indol-2-one.

(IV) : $R_1$ = Cl; $R_2$ = 6-Cl ; $R_3$ = $OCH_3$ ; $R_4$ = H ; Hal = Cl.

A) 5,6-Dichloro-1*H*-indole-2,3-dione.

On prépare ce composé selon le mode opératoire décrit dans J. Am. Chem. Soc., 1946, 68, 2697-2703 ou selon le mode opératoire décrit dans J. Org. Chem., 1952, 17, 149-156.

B) 5,6-Dichloro-3-hydroxy-3-(2-méthoxyphényl)-1,3-dihydro-2*H*-indol-2-one.

A une suspension de 0,72 g de magnésium dans 15 ml d'éther contenant quelques cristaux d'iode, on ajoute, en goutte à goutte, 5,57 g de 1-bromo-2-méthoxybenzène, en maintenant le reflux lorsque celui-ci a démarré. A la fin de l'addition on chauffe 2 heures à reflux. On ajoute ensuite une suspension de 2,7 g de 5,6-dichloro-1*H*-indole-2,3-dione dans 30 ml de THF et chauffe à reflux pendant 30 minutes. Après refroidissement à TA, on verse le mélange réactionnel sur un mélange eau/glace/HCl concentré, extrait à l'AcOEt, sèche la phase organique sur $Na_2SO_4$ et évapore sous vide le solvant. On triture le résidu dans l'éther iso à chaud, essore le précipité formé et le lave à l'éther. On obtient 3 g du produit attendu.

C) 3,5,6-Trichloro-3-(2-méthoxyphényl)-1,3-dihydro-2*H*-indol-2-one.

On refroidit au bain de glace une suspension de 1,5 g du composé obtenu à l'étape précédente dans 30 ml de DCM, ajoute 0,56 ml de pyridine puis 0,5 ml de chlorure de thionyle. Après 1 heure sous agitation à TA, on dilue le mélange réactionnel par ajout de DCM, lave la phase organique à l'eau jusqu'à pH neutre, sèche sur $Na_2SO_4$ et évapore sous vide le solvant. On obtient 1,5 g du produit attendu sous forme de mousses que l'on utilise tel quel.

Préparation 1.3

3,5-Dichloro-3-(2-méthoxyphényl)-6-méthyl-1,3-dihydro-2*H*-indol-2-one.

(IV) : $R_1$ = Cl ; $R_2$ = 6-$CH_3$ ; $R_3$ = $OCH_3$ ; $R_4$ = H ; Hal = Cl.

A) 2-(2-méthoxyphényl)-2-oxoacétate d'éthyle.

On refroidit à -70°C, sous atmosphère d'argon, une solution de 27 g de 1-bromo-2-méthoxybenzène dans 270 ml d'éther, ajoute, goutte à goutte, 90 ml d'une solution 1,6 M de n-butyllithium dans le pentane, puis laisse 45 minutes sous agitation. On ajoute rapidement 78 ml d'oxalate de diéthyle et laisse sous agitation en laissant remonter la température à TA. Après 1 heure d'agitation à TA, on ajoute au mélange réactionnel une solution saturée de $NH_4Cl$, décante, extrait la phase aqueuse à l'éther, lave les phases organiques jointes à l'eau, par une solution saturée de NaCl, sèche sur $Na_2SO_4$ et évapore les solvants sous vide. On élimine l'oxalate de diéthyle en excès par distillation sous vide (Eb = 87°C sous 2000 Pa). On chromatographie le produit résultant sur gel de silice en éluant par le mélange DCM/hexane (50/50 ; v/v) puis au DCM. Le produit obtenu est purifié par distillation sous vide. On obtient 13 g du produit attendu, Eb = 110°C sous 3 Pa.

B) 5-Chloro-3-hydroxy-3-(2-méthoxyphényl)-6-méthyl-1,3-dihydro-2*H*-indol-2-one.

a) 4-Chloro-3-méthylphénylcarbamate de *tert*-butyle.
On laisse 24 heures sous agitation à TA un mélange de 10 g de 4-chloro-3-méthylaniline et 15,26 g de di-*tert*-butyldicarbonate dans 50 ml de dioxane. On concentre sous vide le mélange réactionnel et chromatographie le résidu sur gel de silice en éluant par le gradient du mélange DCM/hexane de (50/50 ; v/v) à (70/30 ; v/v). On obtient 5,6 g du produit attendu que l'on utilise tel quel.

b) On refroidit à -70°C, sous atmosphère d'argon, une solution de 5 g de 4-chloro-3-méthylphénylcarbamate de *tert*-butyle dans 45 ml d'éther, ajoute, goutte à goutte, 30 ml d'une solution 1,5M de *tert*-butyllithium dans le pentane, laisse 1 heure sous agitation en faisant remonter la température à -10°C et laisse 1 heure 45 minutes sous agitation à -10°C. On refroidit le mélange réactionnel à -70°C, ajoute, goutte à goutte, une solution de 5 g du composé obtenu à l'étape A dans 25 ml de THF, laisse 1 heure sous agitation en laissant remonter la température à -30°C puis une nuit en laissant remonter la température à TA. On ajoute une solution saturée de $NH_4Cl$ au mélange réactionnel, évapore le THF, extrait trois fois la phase aqueuse résultante à l'AcOEt, lave la phase organique à l'eau, par une solution saturée de NaCl, sèche sur $Na_2SO_4$, évapore partiellement le solvant et essore le produit cristallisé. On obtient 2,6 g du produit attendu, F = 254-256°C.

C) 3,5-Dichloro-3-(2-méthoxyphényl)-6-méthyl-1,3-dihydro-2*H*-indol-2-one.

On refroidit à 0°C un mélange de 1,3 g du composé obtenu à l'étape B dans 30 ml de DCM, ajoute 0,5 ml de pyridine puis 0,763 g de chlorure de thionyle et laisse 2 heures sous agitation après avoir laissé la température remonter à TA. On ajoute de l'eau et du DCM au mélange réactionnel, après décantation lave quatre fois la phase organique à l'eau, sèche sur $Na_2SO_4$, et évapore sous vide le solvant.On obtient le produit attendu sous forme de mousse que l'on utilise tel quel à la Préparation 3.7.

Préparation 1.4

3-Bromo-5-chloro-3-(2-chlorophényl)-1,3-dihydro-2*H*-indol-2-one.

(IV) : $R_1$ = Cl ; $R_2$ = H ; $R_3$ = Cl ; $R_4$ = H ; Hal = Br.

On prépare ce composé selon les modes opératoires décrits dans WO 95/18105 aux étapes A), B) et C) de la Préparation 2.

Préparation 1.5

3,5-Dichloro-3-(2,3-diméthoxyphényl)-1,3-dihydro-2*H*-indol-2-one.

(IV) : $R_1$ = Cl ; $R_2$ = H ; $R_3$ = $OCH_3$ ; $R_4$ = 3-$OCH_3$ ; Hal = Cl.

A) 2-(2,3-Diméthoxyphényl)-2-oxoacétate d'éthyle.

On refroidit à -40°C un mélange de 27,6 g de 1,2-diméthoxybenzène dans 160 ml d'éther, ajoute, goutte à goutte, 250 ml d'une solution 1,6 M de n-butyllithium dans l'hexane, puis laisse 24 heures sous agitation en laissant remonter la température à TA. On refroidit le mélange réactionnel à -20°C, ajoute rapidement 136 ml d'oxalate de diéthyle et laisse sous agitation en laissant remonter la température à TA. Après 30 minutes d'agitation à TA, on verse le mélange réactionnel sur une solution saturée de $NH_4Cl$, décante, extrait la phase aqueuse à l'éther, lave les phases organiques jointes deux fois à l'eau, sèche sur $Na_2SO_4$ et évapore sous vide les solvants. On élimine l'oxalate de diéthyle en excès par distillation sous vide (Eb=90°C sous 2400Pa). On chromatographie le produit brut résultant sur gel de silice en éluant par le mélange heptane/éther iso (90/10 ; v/v). On obtient 25 g du produit attendu que l'on utilise tel quel à l'étape suivante.

B) 5-Chloro-3-hydroxy-3-(2,3-diméthoxyphényl)-1,3-dihydro-2*H*-indol-2-one.

a) 4-Chlorophénylcarbamate de *tert*-butyle.
On laisse 24 heures sous agitation à TA un mélange de 12,7 g de 4-chloroaniline et 22 g de di-*tert*-butyldicarbonate dans 60 ml de dioxane. On concentre sous vide le mélange réactionnel, reprend le résidu au pentane, essore le précipité formé et le sèche. On obtient 22,5 g du produit attendu.

b) On refroidit à -40°C, sous atmosphère d'azote sec, un mélange de 11,4 g de 4-chlorophénylcarbamate de *tert*-butyle dans 100ml d'éther, ajoute, goutte à goutte, 80 ml d'une solution 1,5 M de *tert*-butyllithium dans le pentane et laisse 3 heures sous agitation à -20°C. On refroidit le mélange réactionnel à -40°C, ajoute, en une heure, une solution de 14 g du composé obtenu à l'étape A dans 50 ml de THF et laisse 4 jours sous agitation à TA. On verse le mélange réactionnel sur une solution saturée de $NH_4Cl$, essore le précipité formé et le sèche. On obtient 10,2 g du produit attendu que l'on utilise tel quel à l'étape suivante.

C) 3,5-Dichloro-3-(2,3-diméthoxyphényl)-1,3-dihydro-2*H*-indol-2-one.

A un mélange de 3,35 g du composé obtenu à l'étape B dans 30 ml de DCM on ajoute, à 0°C, 1,27 ml de pyridine puis 1,2 ml de chlorure de thionyle et laisse 2 heures sous agitation. On lave le mélange réactionnel l'eau, sèche sur $Na_2SO_4$ et évapore sous vide le solvant. On obtient le produit attendu que l'on utilise tel quel à la Préparation 3.9.

Préparation 1.6

3,5-Dichloro-3-(2,4-diméthoxyphényl)-1,3-dihydro-2*H*-indol-2-one.

(IV) : $R_1$ = Cl ; $R_2$ = H ; $R_3$ = $OCH_3$ ; $R_4$ = 4-$OCH_3$ ; Hal = Cl.

A) 5-Chloro-3-hydroxy-3-(2,4-diméthoxyphényl)-1,3-dihydro-2*H*-indol-2-one

On prépare une solution de bromure de 2,4-diméthoxyphénylmagnésium à partir de 2,2 g de magnésium dans 10 ml de TFH et d'une solution de 18 g de 1-bromo-2,4-diméthoxybenzène dans 40 ml de THF. On ajoute, en goutte à goutte, cette solution à un mélange de 5 g de 5-chloro-1*H*-indole-2,3-dione dans 50 ml de THF à une température de 30°C, puis chauffe 2 heures à reflux. On refroidit le mélange réactionnel à TA, le verse sur une solution saturée de $NH_4Cl$, extrait à l'AcOEt, lave la phase organique à l'eau, sèche sur $Na_2SO_4$ et évapore sous vide le solvant. On obtient 7,2 g du produit attendu après cristallisation dans l'éther iso à chaud.

B) 3,5-Dichloro-3-(2,4-diméthoxyphényl)-1,3-dihydro-2*H*-indol-2-one.

On refroidit à 0°C un mélange de 3,8 g du composé obtenu à l'étape précédente et 1,35 ml de pyridine dans

80 ml de DCM, ajoute, en goutte à goutte, 1,22 ml de chlorure de thionyle et laisse 30 minutes sous agitation à 0°C. On lave deux fois le mélange réactionnel à l'eau, sèche sur $Na_2SO_4$, concentre de moitié sous vide et utilise cette solution telle quelle à la Préparation 3.10.

Préparation 1.7

3,5-Dichloro-3-(1,3-benzodioxol-4-yl)-1,3-dihydro-2*H*-indol-2-one.

(IV) : $R_1$ = Cl ; $R_2$ = H ; $R_3$ + $R_4$= 2,3-O-$CH_2$-O-; Hal = Cl.

A) 4-Bromo-1,3-benzodioxole

On prépare ce composé selon le procédé décrit dans Tetrahedron Lett., 1995, 36, 6413-6414.

B) 5-Chloro-3-(1,3-benzodioxol-4-yl)-3-hydroxy-1,3-dihydro-2*H*-indol-2-one.

On prépare une solution de bromure de 1,3-benzodioxol-4-ylmagnésium à partir de 0,85 g de magnésium dans 10 ml de THF et d'une solution de 6,7 g du composé obtenu à l'étape précédente dans 40 ml de THF. On ajoute, en goutte à goutte et à une température inférieure à 40°C, cette solution à un mélange de 3 g de 5-chloro-1*H*-indole-2,3-dione dans 50 ml de THF puis laisse une heure sous agitation. On verse le mélange réactionnel sur une solution saturée de $NH_4Cl$, extrait à l'AcOEt, lave la phase organique à l'eau, sèche sur $Na_2SO_4$ et évapore sous vide le solvant. on obtient 1,12 g du produit attendu après cristallisation dans le DCM, F = 271°C.

C) 3,5-Dichloro-3-(1,3-benzodioxol-4-yl)-1,3-dihydro-2*H*-indol-2-one.

A un mélange de 1,1 g du composé obtenu à l'étape précédente et 0,4 ml de pyridine dans 20 ml de DCM, on ajoute, à une température inférieure à 25°C, 0,3 ml de chlorure de thionyle et laisse 30 minutes sous agitation. On lave deux fois le mélange réactionnel à l'eau, sèche la phase organique sur $Na_2SO_4$ et évapore sous vide le solvant. On obtient 0,62 g du produit attendu après cristallisation dans le DCM, F = 241°C.

Préparation 1.8

3-Bromo-5,6-dichloro-3-(2-chlorophényl)-1,3-dihydro-2*H*-indol-2-one.

(IV) : $R_1$ = Cl ; $R_2$ = 6-Cl ; $R_3$ = Cl ; $R_4$ = H ; Hal = Br.

On prépare ce composé selon les modes opératoires décrits dans WO 95/18105 aux étapes A), B) et C) de la Préparation 72.

Préparation 1.9

3,5-Dichloro-3-(2-chlorophényl)-6-méthoxy-1,3-dihydro-2*H*-indol-2-one.

(IV) : $R_1$= Cl ; $R_2$ = 6-$OCH_3$ ; $R_3$ = Cl ; $R_4$ = H ; Hal = Cl.

A) 4-Chloro-3-méthoxyaniline.

On hydrogène dans un appareil de Parr pendant 4 heures, à 35°C et sous une pression de 1,3 bars, un mélange de 36 g de 2-chloro-5-nitroanisole et du nickel de Raney® dans 150 ml de MeOH et 200 ml de THF. On filtre le catalyseur sur Célite® et concentre sous vide le filtrat. On obtient 28 g du produit attendu que l'on utilise tel quel.

B) N-(4-chloro-3-méthoxyphényl)-DL-2-chloromandélamide.

On chauffe à 230°C pendant 4 heures un mélange de 28 g du composé obtenu à l'étape précédente et 33,13 g d'acide DL-2-chloromandélique dans 128 ml de 1,2-dichlorobenzène en éliminant l'eau formée à l'aide d'un appareil de Dean-Stark. On concentre sous vide en partie le mélange réactionnel et laisse en cristallisation. On essore le produit cristallisé formé et le lave à l'éther iso. On obtient 40 g du produit attendu.

C) 5-Chloro-3-(2-chlorophényl)-6-méthoxy-1,3-dihydro-2*H*-indol-2-one.

On ajoute rapidement 40 g du composé obtenu à l'étape précédente à 550 g d'acide polyphosphorique puis on chauffe à 60°C pendant 8 heures et laisse une nuit sous agitation en laissant revenir la température à TA. On ajoute de l'eau glacée au mélange réactionnel, essore le précipité formé et le lave à l'eau. On reprend le précipité dans l'AcOEt, essore le produit blanc obtenu après trituration et le lave à l'éther iso. On obtient 17,2 g du produit attendu, F = 243-247°C.

D) 5-Chloro-3-(2-chlorophényl)-3-hydroxy-6-méthoxy-1,3-dihydro-2*H*-indol-2-one.

A une solution de 17,2 g du composé obtenu à l'étape précédente dans 220 ml de THF, on ajoute à TA, sous atmosphère d'argon, 2,56 g d'hydrure de sodium à 60 % dans l'huile. Après cessation du dégagement gazeux, on ajoute 6,85 g de diméthyldisulfure, fait barboter de l'air dans le mélange réactionnel et laisse 72 heures sous agitation à TA. On ajoute de l'eau au mélange réactionnel, évapore sous vide le THF, extrait la phase aqueuse restante à l'AcOEt, lave la phase organique à l'eau, par une solution saturée de NaCl, sèche sur $Na_2SO_4$ et évapore sous vide le solvant. On dissout le produit obtenu dans du DCM, concentre en partie le solvant, laisse en cristallisation et essore le produit cristallisé formé. On obtient 6 g du produit attendu, F = 237-240°C.

E) 3,5-Dichloro-3-(2-chlorophényl)-6-méthoxy-1,3-dihydro-2*H*-indol-2-one.

On refroidit au bain de glace une suspension de 3 g du composé obtenu à l'étape précédente dans 90 ml de DCM, ajoute 1,2 ml de pyridine puis 0,96 ml de chlorure de thionyle et laisse 30 minutes sous agitation. On lave le mélange réactionnel à l'eau jusqu'à pH neutre, sèche la phase organique sur $Na_2SO_4$ et concentre sous vide de moitié la solution. On obtient une solution du produit attendu que l'on utilise directement à la Préparation 3.13.

Préparation 1.10

3,6-Dichloro-3-(2-méthoxyphényl)-5-méthyl-1,3-dihydro-2*H*-indol-2-one.

(IV) : $R_1 = CH_3$ ; $R_2$ = 6-Cl ; $R_3 = OCH_3$ ; $R_4$ = H ; Hal = Cl.

A) 6-Chloro-5-méthyl-3-méthylthio-1,3-dihydro-2*H*-indol-2-one et 4-chloro-5-méthyl-3-méthylthio-1,3-dihydro-2*H*-indol-2-one.

Dans 320 ml de DCM refroidi à -70°C, on introduit 8,5 ml de chlore, puis ajoute, en 20 minutes et à -70°C, une solution de 24 ml de méthylthioacétate d'éthyle dans 60 ml de DCM et laisse 15 minutes sous agitation à -70°C. On ajoute ensuite, à -70°C et en 30 minutes, une solution de 52,64 g de 3-chloro-4-méthylaniline dans 100 ml de DCM et laisse 1 heure 45 minutes sous agitation à -70°C. On ajoute enfin, à -70°C, 41,3 ml de triéthylamine et laisse 1 heure sous agitation en laissant remonter la température à TA. On lave le mélange réactionnel deux fois par 250 ml d'eau, sèche la phase organique sur $MgSO_4$ et évapore sous vide le solvant. On reprend le résidu dans un mélange de 600 ml d'éther et 130 ml d'HCl 2N et laisse 72 heures sous agitation à TA. On filtre un insoluble, décante le filtrat, lave deux fois la phase organique à l'eau, sèche sur $MgSO_4$ et évapore sous vide le solvant. On chromatographie le résidu sur gel de silice en éluant au DCM puis par le mélange DCM/AcOEt (85/15 ; v/v). On rechromatographie le mélange obtenu sur gel de silice en éluant au DCM puis par le mélange DCM/AcOEt (95/5 ; v/v). On sépare les deux isomères :

- l'isomère le moins polaire qui est le 6-chloro-5-méthyl-3-méthylthio-1,3-dihydro-2*H*-indol-2-one et obtient 1,16 g.
- l'isomère le plus polaire qui est le 4-chloro-5-méthyl-3-méthylthio-1,3-dihydro-2*H*-indol-2-one et obtient 0,72 g.

B) 6-Chloro-5-méthyl-1*H*-indole-2,3-dione.

On chauffe à reflux pendant 1 heure un mélange de 1,16 g de 6-chloro-5-méthyl-3-méthylthio-1,3-dihydro-2*H*-indol-2-one obtenu à l'étape précédente et 0,681 g de N-chlorosuccinimide dans 100 ml de tétrachlorure de carbone. On concentre sous vide le mélange réactionnel, reprend le résidu dans un mélange de 80 ml de THF et 20 ml d'eau puis chauffe à reflux pendant 16 heures. On évapore sous vide le THF, extrait la phase aqueuse restante à l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le gradient du mélange DCM/AcOEt jusqu'à (85/15 ; v/v). On obtient 0,793 g du produit attendu, F = 264°C.

C) 6-Chloro-3-hydroxy-3-(2-méthoxyphényl)-5-méthyl-1,3-dihydro-2*H*-indol-2-one.

On prépare une solution de bromure de 2-méthoxyphénylmagnésium à partir de 0,687 g de magnésium dans 1,5 ml d'éther et d'une solution de 5,35 g de 1-bromo-2-méthoxybenzène dans 7,55 ml d'éther. On ajoute, en goutte à goutte, sous atmosphère d'argon, cette solution à un mélange de 1,4 g du composé obtenu à l'étape précédente dans 14 ml de THF préalablement refroidi au bain de glace, puis laisse sous agitation en laissant remonter la température à TA. Après 1 heure d'agitation à TA, on verse lentement le mélange réactionnel sur une solution saturée de $NH_4Cl$, évapore le THF sous vide, extrait à l'AcOEt, lave la phase organique à l'eau, par une solution saturée de NaCl, sèche sur $Na_2SO_4$ et évapore sous vide l'AcOEt. On chromatographie le résidu sur gel de silice en éluant au DCM puis par le mélange DCM/MeOH (98/2 ; v/v). On obtient 1,6 g du produit attendu après cristallisation dans le mélange THF/MeOH, F = 266°C.

D) 3,6-Dichloro-3-(2-méthoxyphényl)-5-méthyl-1,3-dihydro-2*H*-indol-2-one.

On refroidit au bain de glace une suspension de 0,913 g du composé obtenu à l'étape précédente dans 10 ml de DCM, ajoute 0,36 ml de pyridine puis 0,33 ml de chlorure de thionyle et laisse 20 minutes sous agitation. On dilue le mélange réactionnel par ajout de 50 ml de DCM, lave trois fois la phase organique à l'eau, sèche sur $Na_2SO_4$ et évapore le solvant sous vide. On obtient 0,5 g du produit attendu.

Préparation 1.11

3,4-Dichloro-3-(2-méthoxyphényl)-5-méthyl-1,3-dihydro-2*H*-indol-2-one.

(IV) : $R_1 = CH_3$ ; $R_2$ = 4-Cl ; $R_3 = OCH_3$ ; $R_4$ = H ; Hal = Cl.

A) 4-Chloro-5-méthyl-1*H*-indole-2,3-dione

On prépare ce composé selon le mode opératoire décrit à l'étape B de la Préparation 1.10 à partir de 0,72 g de 4-chloro-5-méthyl-3-méthylthio-1,3-dihydro-2*H*-indol-2-one, 0,422 g de N-chlorosuccinimide dans 72 ml de tétrachlorure de carbone, puis de 58 ml de THF et 14 ml d'eau. On chromatographie le produit obtenu sur gel de silice en éluant au DCM puis par le gradient du mélange DCM/AcOEt jusqu'à (90/10 ; v/v). On obtient 0,5 g du produit attendu.

B) 4-Chloro-3-hydroxy-3-(2-méthoxyphényl)-5-méthyl-1,3-dihydro-2*H*-indol-2-one.

A une suspension de 0,638 g de magnésium dans 1,5 ml d'éther, on ajoute, en goutte à goutte, une solution de 5 g de 1-bromo-2-méthoxybenzène dans 7 ml d'éther jusqu'à ce que la réaction démarre puis poursuit l'addition en maintenant le reflux. A la fin de l'addition, on chauffe à 30°C pendant 20 minutes. On ajoute, en goutte à goutte et sous atmosphère d'argon, cette solution à une suspension de 1,3 g du composé obtenu à l'étape précédente dans 13 ml de THF préalablement refroidi au bain de glace puis laisse sous agitation en laissant remonter la température à TA. Après 1 heure à TA, on verse le mélange réactionnel sur une solution saturée de $NH_4Cl$, évapore sous vide le THF, extrait à l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le mélange DCM/MeOH (98/2 ; v/v). On obtient 0,846 g du produit attendu après cristallisation dans le mélange THF/MeOH, F = 262-263°C.

C) 3,4-Dichloro-3-(2-méthoxyphényl)-5-méthyl-1,3-dihydro-2*H*-indol-2-one.

On refroidit à 0°C une suspension de 1,5 g du composé obtenu à l'étape précédente dans 30 ml de DCM, ajoute 0,6 ml de pyridine puis 0, 54 ml de chlorure de thionyle et laisse 45 minutes sous agitation. On dilue le mélange réactionnel par ajout de 15 ml de DCM, lave trois fois la phase organique à l'eau, sèche sur $MgSO_4$ et évapore sous vide le solvant. On obtient 1 g du produit attendu.

Préparations des composés de formule (V).

Préparation 2.1

Chlorhydrate de (3S)-N,N-diméthyl-1,2,3,4-tétrahydroisoquinoléine-3-carboxamide.

(V), HCl :

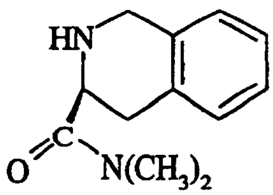

A) (3S)-N,N-Diméthyl-2-(*tert*-butoxycarbonyl)-1,2,3,4-tétrahydroisoquinoléine-3-carboxamide.

On refroidit à 0°C une solution de 5 g d'acide (3S)-2-(*tert*-butoxycarbonyl)-1,2,3,4-tétrahydroisoquinoléine-3-carboxylique (commercial) dans 50 ml de DCM, ajoute 5,45 g de triéthylamine puis 8 g de BOP. Puis on ajoute de la diméthylamine gaz par barbotage pendant 5 minutes et laisse 18 heures sous agitation à TA. On reprend le mélange réactionnel par un mélange eau/DCM, après décantation lave la phase organique par une solution à 5 % de $KHSO_4$, par une solution à 5 % de $Na_2CO_3$, sèche sur $Na_2SO_4$ et évapore sous vide le solvant. On chromatographie le résidu sur gel de silice en éluant par le mélange DCM/AcOEt (70/30 ; v/v). On obtient 4 g du produit attendu sous forme d'huile qui cristallise.

B) Chlorhydrate de (3S)-N,N-diméthyl-1,2,3,4-tétrahydroisoquinoléine-3-carboxamide.

On laisse 2 heures sous agitation à TA un mélange de 4 g du composé obtenu à l'étape précédente dans 50 ml d'une solution 4N d'HCl dans le dioxane. On concentre sous vide le mélange réactionnel, reprend le résidu par un mélange DCM/éther iso et essore le précipité formé. On obtient 4,9 g du produit attendu.

Préparation 2.2

(1S)-N,N-Diméthyl-1,2,3,4-tétrahydroisoquinoléine-1-carboxamide.

(V) :

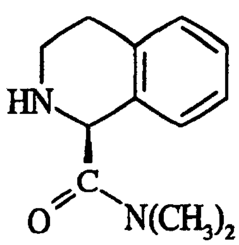

A) (1S)-N,N-Diméthyl-2-(9-fluorénylméthoxycarbonyl)-1,2,3-4-tétrahydroiso quinoléine-1-carboxamide.

A une solution de 5 g d'acide (1S)-2-(9-fluorénylméthoxycarbonyl)-1,2,3-4-tétrahydroisoquinoléine-1-carboxylique (commercial) dans 100 ml de DCM, on ajoute 1,61 g de diisopropyléthylamine, 5,53 g de BOP, puis 5 ml d'une solution 5,6M de diméthylamine dans l'éthanol et laisse 3 heures sous agitation à TA. On reprend le mélange réactionnel par un mélange eau/DCM, après décantation lave la phase organique par une solution à 5 % de $KHSO_4$, sèche sur $Na_2SO_4$ et évapore sous vide le solvant. On chromatographie le résidu sur gel de silice en éluant par le mélange DCM/AcOEt (70/30 ; v/v). On obtient 4,5 g du produit attendu sous forme d'huile.

B) (1S)-N,N-Diméthyl-1,2,3,4-tétrahydroisoquinoléine-1-carboxamide.

On prépare ce composé en utilisant la méthode décrite dans Synthetic Communications, 1994, 24 (2), 187-195. A une solution de 4,5 g du composé obtenu à l'étape précédente dans 50 ml de DMF, on ajoute, à TA et sous atmosphère d'azote, 4 g de fluorure de potassium et 0,4 g d'éther couronne 18-Crown-6 et laisse 18 heures sous agitation à TA. On concentre sous vide le mélange réactionnel, reprend le résidu à l'éther, filtre un insoluble et concentre sous vide le filtrat. On chromatographie l'huile résultante sur gel de silice en éluant par le mélange DCM/MeOH (80/20 ; v/v). On obtient 1,6 g du produit attendu sous forme d'huile.

Préparation 2.3

(2S)-N,N-Diméthyl-2,3-dihydro-1*H*-indole-2-carboxamide.

(V) : 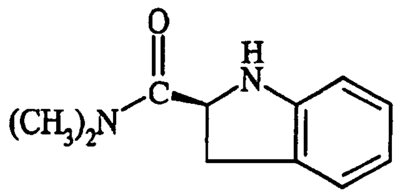

A) (2S)-N,N-Diméthyl-1-(*tert*-butoxycarbonyl)-2,3-dihydro-1*H*-indole-2-carboxamide.

On refroidit au bain de glace un mélange de 5 g d'acide (2S)-1-(*tert*-butoxycarbonyl)-2,3-dihydro-1*H*-indole-2-carboxylique (commercial) et de 5,8 g de triéthylamine dans 100 ml de DCM, ajoute 8,4 g de BOP et laisse 15 minutes sous agitation. Puis on ajoute de la diméthylamine gaz par barbotage pendant 10 minutes et laisse 3 heures sous agitation à TA. On concentre sous vide le mélange réactionnel, extrait le résidu à l'AcOEt, lave la phase organique à l'eau, par une solution à 5 % de $Na_2CO_3$, par une solution à 5 % de $KHSO_4$, sèche sur $Na_2SO_4$ et évapore sous vide le solvant. On chromatographie le résidu sur gel de silice en éluant par le mélange DCM/AcOEt (85/15 ; v/v). On obtient 4,1 g du produit attendu.

B) (2S)-N,N-Diméthyl-2,3-dihydro-1*H*-indole-2-carboxamide.

On laisse 2 heures sous agitation à TA, un mélange de 4 g du composé obtenu à l'étape précédente dans 20 ml de TFA. On concentre sous vide le mélange réactionnel, reprend le résidu par une solution à 5 % de $Na_2CO_3$ jusqu'à pH = 9, extrait à l'éther, sèche la phase organique sur $Na_2SO_4$ et évapore sous vide le solvant. On obtient 2,65 g du produit attendu.

Préparation 2.4

Chlorhydrate de l'ester méthylique de l'acide (3S)-1,2,3,4-tétrahydroisoquinoléine-3-carboxylique.

(V),.HCl : 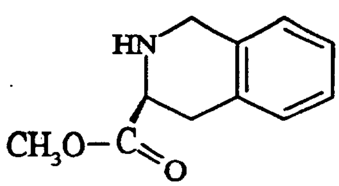

On chauffe à reflux pendant 72 heures un mélange de 5 g de chlorhydrate de l'acide (3S)-1,2,3,4-tétrahydroisoquinoléine-3-carboxylique (commercial) et de 100 ml d'une solution saturée d'acide chlorhydrique dans le MeOH. On concentre sous vide le mélange réactionnel, reprend le résidu au MeOH et évapore sous vide le solvant. On obtient

6 g du produit attendu.

Préparation des composés de formule (II).

Préparation 3.1

(3S)-2-[5-Chloro-3-(2-méthoxyphényl)-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = H ; $R_3$ = $OCH_3$ ; $R_4$ = H ; $R_5$ = $N(CH_3)_2$ ; $R_8$ = H ; $R_9$ = H ; n = 1 ; p = 1.

On laisse 48 heures sous agitation à TA un mélange de 2 g du composé obtenu à la préparation 1.1, 1,71 g du composé obtenu à la Préparation 2.1 et 1,5 g de triéthylamine dans 50 ml de THF. On concentre sous vide le mélange réactionnel, extrait le résidu au DCM, lave la phase organique à l'eau, sèche sur $Na_2SO_4$ et évapore sous vide le solvant. On chromatographie le résidu sur gel de silice en éluant par le mélange DCM/AcOEt (50/50 ; v/v). On obtient 3,6 g du produit attendu sous forme du mélange de diastéréoisomères.

Préparation 3.2

(1S)-2-[5-Chloro-3-(2-méthoxyphényl)-2-oxo-2,3-dihydro-1*H*-indol-3-yl]-N,N-diméthyl-1,2,3,4-tétrahydroisoquinoléine-1-carboxamide, mélange de diastéréoisomères.

(II) : $R_1$ = Cl ; $R_2$ = H ; $R_3$ = $OCH_3$ ; $R_4$ = H ; $R_5$ = $N(CH_3)_2$ ; $R_8$ = H ; $R_9$ = H ; n = 2 ; p = 0.

On prépare ce composé selon le mode opératoire décrit à la Préparation 3.1 à partir de 2,4 g du composé obtenu à la Préparation 1.1, 1,6 g du composé obtenu à la Préparation 2.2 et 1,57 g de triéthylamine dans 50 ml de THF. On obtient 2,5 g du produit attendu sous forme du mélange des diastéréoisomères.

Préparation 3.3

(2S)-1-[5-Chloro-3-(2-méthoxyphényl)-2-oxo-2,3-dihydro-1*H*-indol-3-yl]-N,N-diméthyl-2,3-dihydro-1*H*-indole-2-carboxamide, isomère lévogyre.

(II) : $R_1$ = Cl ; $R_2$ = H ; $R_3$ = $OCH_3$ ; $R_4$ = H ; $R_5$ = $N(CH_3)_2$ ; $R_8$ = H ; $R_9$ = H ; n = 0 ; p = 1.

On laisse 48 heures sous agitation à TA un mélange de 5 g du composé obtenu à la Préparation 1.1, 3 g du composé obtenu à la Préparation 2.3 et 1,62 g de triéthylamine dans 100 ml de THF et 100 ml de chloroforme. On concentre sous vide le mélange réactionnel, extrait le résidu au DCM, lave la phase organique à l'eau, sèche sur $Na_2SO_4$ et évapore sous vide le solvant. On chromatographie le résidu sur gel de silice en éluant par le mélange DCM/AcOEt (50/50 ; v/v). On sépare les diastéréoisomères et recueille le composé le plus polaire. On obtient 2,2 g du produit attendu sous forme de mousses.
$\alpha_D^{20}$ = -515° (c = 0,4 ; chloroforme).

Préparation 3.4

Ester méthylique de l'acide (3S)-2-[5-Chloro-3-(2-méthoxyphényl)-2-oxo-2,3-dihydro-1*H*-indol-3-yl]-1,2,3,4-tétrahydroisoquinoléine-3-carboxylique, mélange de diastéréoisomères.

(II) : $R_1$ = Cl ; $R_2$ = H ; $R_3$ = $OCH_3$ ; $R_4$ = H ; $R_5$ = $OCH_3$ ; $R_8$ = H ; $R_9$ = H ; n = 1 ; p = 1.

On chauffe pendant 2 heures à reflux un mélange de 1,6 g du composé obtenu à la Préparation 1.1, 1,42 g du composé obtenu à la Préparation 2.4 et 1,3 g de triéthylamine dans 20 ml de THF ET 20 ml de chloroforme. On concentre sous vide le mélange réactionnel, extrait le résidu au DCM, lave la phase organique à l'eau, sèche sur $Na_2SO_4$ et évapore sous vide le solvant. On chromatographie le résidu sur gel de silice en éluant au DCM puis par le gradient du mélange DCM/AcOEt de (99/1 ; v/v) à (90/10 ; v/v). On obtient 2,3 g du produit attendu sous forme du mélange de diastéréoisomères.

Préparation 3.5

(3S)-2-[5,6-Dichloro-3-(2-méthoxyphényl)-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = 6-Cl ; $R_3$ = $OCH_3$ ; $R_4$ = H ; $R_5$ = $N(CH_3)_2$ ; $R_8$ = H ; $R_9$ = H ; n = 1 ; p = 1.

A une solution de 1,5 g du composé obtenu à la Préparation 1.2 et 1,5 g du composé obtenu à la Préparation 2.1 (sous forme de base libre) dans 30 ml de THF, on ajoute 0,7 g de triéthylamine et laisse 18 heures sous agitation à TA. On concentre sous vide le mélange réactionnel, extrait le résidu au DCM, lave la phase organique à l'eau, sèche sur $Na_2SO_4$ et évapore sous vide le solvant. On chromatographie le résidu sur gel de silice en éluant par le mélange DCM/AcOEt (60/40 ; v/v). On obtient 1,4 g du produit attendu sous forme du mélange de diastéréoisomères.

Préparation 3.6

(3S)-2-[5-Chloro-3-(2-méthoxyphényl)-6-méthyl-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = 6-$CH_3$ ; $R_3$ = $OCH_3$ ; $R_4$ = H ; $R_5$ = $N(CH_3)_2$ ; $R_8$ = H ; $R_9$ = H ; n = 1 ; p = 1.

A une solution du composé obtenu à la Préparation 1.3 dans 50 ml de THF, on ajoute 1,7 g du composé obtenu à la Préparation 2.1 (sous forme de base libre) puis 0,86 g de triéthylamine et laisse 18 heures sous agitation à TA. On concentre sous vide le mélange réactionnel, extrait le résidu à l'AcOEt, lave la phase organique à l'eau, sèche sur $Na_2SO_4$ et évapore sous vide le solvant. On chromatographie le résidu sur gel de silice en éluant par le mélange DCM/AcOEt (50/50 ; v/v). On obtient 1,5 g du produit attendu sous forme du mélange de diastéréoisomères.

Préparation 3.7

(3S)-2-[5-Chloro-3-(2-chlorophényl)-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = H ; $R_3$ = Cl ; $R_4$ = H ; $R_5$ = $N(CH_3)_2$ ; $R_8$ = H ; $R_9$ = H ; n = 1 ; p = 1.

On laisse 3 heures sous agitation à TA un mélange de 2 g du composé obtenu à la Préparation 1.4, 1,25 g du composé obtenu à la Préparation 2.1 (base libre) et 0,678 g de triéthylamine dans 50 ml de THF. On concentre sous vide le mélange réactionnel, extrait le résidu au DCM, lave la phase organique à l'eau, sèche sur $Na_2SO_4$ et évapore le solvant sous vide. On chromatographie le résidu sur gel de silice en éluant par le mélange DCM/AcOEt (80/20 ; v/v). On obtient 1,7 g du produit attendu sous forme du mélange des diastéréoisomères.

Préparation 3.8

(3S)-2-[5-Chloro-3-(2,3-diméthoxyphényl)-2-oxo-2,3-dihydro-1*H*-indol-3-yl]-N,N-diméthyl-1,2,3,4-tétrahydroisoquinoléine-3-carbaxamide, mélange de diastéréoisomères.

(II) : $R_1$ = Cl ; $R_2$ = H ; $R_3$ = $OCH_3$ ; $R_4$ = 3-$OCH_3$ ; $R_5$ = $N(CH_3)_2$ ; $R_8$ = H ; $R_9$ = H ; n = 1 ; p = 1.

A un mélange du composé obtenu à la Préparation 1.5 et 1,7 g du composé obtenu à la Préparation 2.1 dans 50 ml de THF et 10 ml de DCM, on ajoute 2,63 g de triéthylamine et laisse 72 heures sous agitation à TA. On concentre sous vide, extrait le résidu au DCM, lave la phase organique à l'eau, sèche sur $Na_2SO_4$ et évapore le solvant sous vide. On chromatographie le résidu sur gel de silice en éluant par le mélange DCM/AcOEt (50/50 ; v/v). On obtient 1,2 g du produit attendu sous forme du mélange de diastéréoisomères.

Préparation 3.9

(3S)-2-[5-Chloro-3-(2,4-diméthoxyphényl)-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = H ; $R_3$ = $OCH_3$ ; $R_4$ = 4-$OCH_3$ ; $R_5$ = $N(CH_3)_2$ ; $R_8$ = H ; $R_9$ = H ; n = 1 ; p = 1.

A la solution du composé obtenu à la Préparation 1.6 dans le DCM, on ajoute 3 g du composé obtenu à la Préparation 2.1 (base libre) et 1,6 g de triéthylamine puis laisse 18 heures sous agitation à TA. On dilue le mélange réactionnel par ajout de DCM, lave la phase organique à l'eau, sèche sur $Na_2SO_4$ et évapore le solvant sous vide. On chromatographie le résidu sur gel de silice en éluant par le mélange DCM/AcOEt (50/50 ; v/v). On obtient 2,8 g du produit attendu sous forme du mélange de diastéréosiomères.

Préparation 3.10

(3S)-2-[5-Chloro-3-(1,3-benzodioxol-4-yl)-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = H ; $R_3$ + $R_4$ = 2,3-O-$CH_2$-O-; $R_5$ = $N(CH_3)_2$ ; $R_8$ = H ; $R_9$ = H ; n = 1 ; p = 1.

A une solution de 1,5 g du composé obtenu à la Préparation 1.7 dans 15 ml de THF, on ajoute 0,89 g du composé obtenu à la Préparation 2.1 (base libre) puis 0,52 g de triéthylamine et laisse 18 heures sous agitation à TA. On concentre sous vide, extrait le résidu au DCM, lave la phase organique à l'eau, sèche sur $Na_2SO_4$ et évapore le solvant sous vide. On chromatographie le résidu sur gel de silice en éluant par le mélange DCM/AcOEt (70/30 ; v/v). On obtient 1,5 g du produit attendu sous forme du mélange de diastéréoisomères.

Préparation 3.11

(3S)-2-[5,6-Dichloro-3-(2-chlorophényl)-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = 6-Cl ; $R_3$ = Cl ; $R_4$ = H ; $R_5$ = $N(CH_3)_2$ ; $R_8$ = H ; $R_9$ = H ; n = 1 ; p = 1.

On laisse 18 heures sous agitation à TA un mélange de 2,66 g du composé obtenu à la Préparation 1.8, 2,1 g du composé obtenu à la Préparation 2.1 (base libre) et 1 g de triéthylamine dans 100 ml de DCM. On lave le mélange réactionnel à l'eau, sèche la phase organique sur $Na_2SO_4$ et évapore le solvant sous vide. On chromatographie le résidu sur gel de silice en éluant par le mélange DCM/AcOEt (80/20 ; v/v). On obtient 1,1 g du produit attendu sous forme du mélange de diastéréoisomères.

Préparation 3.12

(3S)-2-[5-Chloro-3-(2-chlorophényl)-6-méthoxy-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1$ = Cl ; $R_2$ = 6-$OCH_3$ ; $R_3$ = Cl ; $R_4$ = H ; $R_5$ = $N(CH_3)_2$ ; $R_8$ = H ; $R_9$ = H ; n = 1 ; p = 1.

A la solution du composé obtenu à la Préparation 1.9 dans le DCM on ajoute 2,26 g du composé obtenu à la Préparation 2.1 (base libre) et 1,4 g de triéthylamine et laisse 18 heures sous agitation à TA. On dilue le mélange réactionnel au DCM, lave la phase organique à l'eau, sèche sur $Na_2SO_4$ et évapore le solvant sous vide. On chromatographie le résidu sur gel de silice en éluant par le mélange DCM/AcOEt (50/50 ; v/v). On obtient 1,3 g du produit attendu sous forme du mélange de diastéréoisomères.

Préparation 3.13

(3S)-2-[4-Chloro-3-(2-méthoxyphényl)-5-méthyl-2-oxo-2,3-dihydro-1*H*-indol-3-yl]-N,N-diméthyl-1,2,3,4-tétrahydroisoquinoléine-3-carboxamide, mélange de diastéréoisomères.

(II) : $R_1 = CH_3$ ; $R_2$ = 4-Cl ; $R_3 = OCH_3$ ; $R_4$ = H ; $R_5 = N(CH_3)_2$ ; $R_8$ = H ; $R_9$ = H ; n = 1 ; p = 1.

A une solution de 1 g du composé obtenu à la Préparation 1.11 dans 50 ml de THF, on ajoute 1,5 g du composé obtenu à la Préparation 2.1 (base libre) et 1 g de triéthylamine et chauffe à reflux pendant 18 heures. On concentre sous vide le mélange réactionnel, extrait le résidu au DCM, lave la phase organique à l'eau, sèche sur $Na_2SO_4$ et évapore le solvant sous vide. On chromatographie le résidu sur gel de silice en éluant par le mélange DCM/AcOEt (50/50 ; v/v). On obtient 2 g du produit attendu sous forme du mélange de diastéréoisomères.

EXEMPLE 1 et 2

(3S)-2-[5-Chloro-1-[(2,4-diméthoxyphényl)sulfonyl]-3-(2-méthoxyphényl)-2-oxo-2,3-dihydro-1*H*-indol-3-yl]-N,N-diméthyl-1,2,3,4-tétrahydroisoquinoléine-3-carboxamide, isomère A et isomère B.

(Ia) : $R_1$ = Cl ; $R_2$ = H ; $R_3 = OCH_3$ ; $R_4$ = H ; $R_5 = N(CH_3)_2$ ; $R_6 = 2\text{-}OCH_3$ ; $R_7 = OCH_3$ ; $R_8$ = H ; $R_9$ = H ; n = 1 ;p = 1.

A une solution de 1,4 g du composé obtenu à la Préparation 3.1 dans 20 ml de DMF, on ajoute, à TA et sous atmosphère d'azote, 0,123 g d'hydrure de sodium à 60 % dans l'huile et laisse 15 minutes sous agitation. On ajoute ensuite 0,7 g de chlorure de 2,4-diméthoxybenzènesulfonyle et laisse 3 heures sous agitation à TA. On verse le mélange réactionnel dans l'eau, extrait à l'AcOEt, sèche la phase organique sur $Na_2SO_4$ et évapore sous vide le solvant. On chromatographie le résidu sur gel de silice en éluant par le mélange DCM/AcOEt (90/10 ; v/v). On sépare les diastéréoisomères :

- le moins polaire, isomère A : composé de l'Exemple 1 que l'on cristallise dans l'heptane, F =186°C.
  $\alpha_D^{20}$ = +138° (c = 0,6 ; chloroforme).

- le plus polaire, isomère B : composé de l'Exemple 2 que l'on cristallise dans l'heptane et obtient 0,84 g, F = 160°C.
  $\alpha_D^{20}$ = -263° (c = 0,22 ; chloroforme).

RMN $^1H$ : DMSO-$d_6$ : δ (ppm) : 2,4 : se : 6H ; 2,9 : mt : 2H ; 3,1 : s : 3H ; 3,6 et 3,8 : 2se : 6H ; 3,5 et 3,8 : 2mt : 2H ; 3,7 : d : 1H ; 6,5 : s : 1H ; 6,7 : dd : 1H ; 6,8 à 7,1 : m : 7H ; 7,3 : mt : 1H ; 7,4 : dd : 1H ; 7,6 : d : 1H ; 7,8 : d : 1H ; 8,0 : d : 1H.

EXEMPLE 3

(1S)-2-[5-Chloro-1-[(2,4-diméthoxyphényl)sulfonyl]-3-(2-méthoxyphényl)-2-oxo-2,3-dihydro-1*H*-indol-3-yl]-N,N-diméthyl-1,2,3,4-tétrahydroisoquinoléine-1-carboxamide, isomère lévogyre.

(Ia) : $R_1$ = Cl ; $R_2$ = H ; $R_3 = OCH_3$ ; $R_4$ = H ; $R_5 = N(CH_3)_2$ $R_6 = 2\text{-}OCH_3$ ; $R_7 = OCH_3$ ; $R_8$ = H ; $R_9$ = H ; n = 2 ;p = 0.

A une solution de 2,5 g du composé obtenu à la Préparation 3.2 dans 20 ml de DMF, on ajoute, à TA et sous atmosphère d'azote, 0,225 g d'hydrure de sodium à 60 % dans l'huile et laisse 30 minutes sous agitation. On ajoute ensuite 1,25 g de chlorure de 2,4-diméthoxybenzènesulfonyle et laisse 1 heure sous agitation. On verse le mélange réactionnel dans l'eau, extrait à l'AcOEt, lave la phase organique à l'eau, sèche sur $Na_2SO_4$ et évapore sous vide le solvant. On chromatographie le résidu sur gel de silice en éluant par le mélange DCM/AcOEt (90/10 ; v/v). On sépare les diastéréoisomères et recueille le composé le plus polaire. On obtient 1,2 g du produit attendu après cristallisation dans l'éther iso, F = 263°C.
$\alpha_D^{20}$ = -262° (c = 0,4 ; chloroforme).
RMN $^1H$ : DMSO-$d_6$ : δ (ppm) : 2,0 à 3,0 : m+2s : 8H ; 3,2 à 4,2 : mt+s : 11H ; 5,2 et 5,4 : 2s : 1H ; 6,4 à 8,0 : mt : 14H.

EXEMPLE 4

(2S)-1-[5-Chloro-1-[(2,4-diméthoxyphényl)sulfonyl]-3-(2-méthoxyphényl)-2-oxo-2,3-dihydro-1*H*-indol-3-yl]-N,N-diméthyl-2,3-dihydro-1*H*-indole-2-carboxamide, isomère lévogyre.

(Ia) : $R_1$ = Cl ; $R_2$ = H ; $R_3$ = $OCH_3$ ; $R_4$ = H ; $R_5$ = $N(CH_3)_2$ ; $R_6$ = 2-$OCH_3$ ; $R_7$ = $OCH_3$ ; $R_8$ = H ; $R_9$ = H; n = 0 ;p = 1.

A une solution de 0,7 g du composé obtenu à la Préparation 3.3 dans 20 ml de DMF, on ajoute, à TA et sous atmosphère d'azote, 0,066 g d'hydrure de sodium à 60 % dans l'huile et laisse 15 minutes sous agitation. On ajoute ensuite 0,36 g de chlorure de 2,4-diméthoxybenzènesulfonyle et laisse 2 heures sous agitation à TA. On verse le mélange réactionnel dans l'eau, extrait à l'AcOEt, lave la phase organique à l'eau, sèche sur $Na_2SO_4$ et évapore sous vide le solvant. On chromatographie le résidu sur gel de silice en éluant par le mélange DCM/AcOEt (70/30 ; v/v). On obtient 0,58 g du produit attendu après cristallisation dans l'éther, F = 167°C.
$\alpha_D^{20}$ = -305° (c = 0,18 ; choroforme).
RMN $^1H$ : DMSO-$d_6$ : δ (ppm) : 2,4 et 2,7 : 2s : 6H ; 2,6 et 3,6 : 2mt : 2H ; 3,1 : s : 3H ; 3,8 : 2s : 6H ; 5,2 : dd : 1H ; 6,4 : d : 1H ; 6,5 : t : 1H ; 6,5 à 7,6 : m : 10H ; 7,8 : d : 1H ; 8,0 : d : 1H.

EXEMPLE 5

Ester méthylique de l'acide (3S)-2-[5-Chloro-1-[(2,4-diméthoxyphényl)sulfonyl]-3-(2-méthoxyphényl)-2-oxo-2,3-dihydro-1*H*-indol-3-yl]-1,2,3,4-tétrahydroisoquinoléine-3-carboxylique, isomère lévogyre.

(Ia) : $R_1$ = Cl ; $R_2$ = H ; $R_3$ = $OCH_3$ ; $R_4$ = H ; $R_5$ = $OCH_3$ ; $R_6$ = 2-$OCH_3$ ; $R_7$ = $OCH_3$ ; $R_8$ = H ; $R_9$ = H ; n = 1 ;p = 1.
A une solution de 2,3 g du composé obtenu à la Préparation 3.4 dans 20 ml de DMF, on ajoute, à TA et sous atmosphère d'azote, 0,208 g d'hydrure de sodium à 60 % dans l'huile et laisse 15 minutes sous agitation. On ajoute ensuite 1,17 g de chlorure de 2,4-diméthoxybenzènesulfonyle et laisse 2 heures sous agitation à TA. On verse le mélange réactionnel dans l'eau, extrait à l'AcOEt, lave la phase organique à l'eau, sèche sur $Na_2SO_4$ et évapore sous vide le solvant. On chromatographie le résidu sur gel de silice en éluant par le mélange DCM/hexane (70/30 ; v/v) puis par le mélange DCM/AcOEt (95/5 ; v/v). On sépare les diastéréoisomères et recueille le composé le plus polaire. On obtient 1,1 g du produit attendu après cristallisation dans l'hexane, F = 170°C.
$\alpha_D^{20}$ = - 220° (c = 0,2 ; chloroforme).
RMN $^1H$ : DMSO-$d_6$ : δ (ppm) : 2,8 à 4,4 : m : 17H ; 6,0 à 7,0 : m : 9H ; 7,2 : t : 1H ; 7,4 : dd : 1H ; 7,6 : d : 1H ; 7,8 : d : 1H ; 8,0 : d : 1H.

EXEMPLE 6

(3S)-2-[5,6-Dichloro-1-[(2,4-diméthoxyphényl)sulfonyl]-3-(2-méthoxyphényl)-2-oxo-2,3-dihydro-1*H*-indol-3-yl]-N,N-diméthyl-1,2,3,4-tétrahydroisoquinoléine-3-carboxamide, isomère lévogyre.

(Ia) : $R_1$ = Cl ; $R_2$ = 6-Cl ; $R_3$ = $OCH_3$ ; $R_4$ = H ; $R_5$ = $N(CH_3)_2$ ; $R_6$ = 2-$OCH_3$ ; $R_7$ = $OCH_3$ ; $R_8$ = R ; $R_9$ = H ; n = 1 ;p = 1.
A une solution de 1,35 g du composé obtenu à la Préparation 3.5 dans 10 ml de DMF, on ajoute, à TA et sous atmosphère d'azote, 0,111 g d'hydrure de sodium à 60 % dans l'huile et laisse 15 minutes sous agitation. On ajoute ensuite 0,624 g de chlorure de 2,4-diméthoxybenzènesulfonyle et laisse 2 heures sous agitation à TA. On verse le mélange réactionnel dans l'eau, extrait à l'AcOEt, sèche la phase organique sur $Na_2SO_4$ et évapore sous vide le solvant. On chromatographie le résidu sur gel de silice en éluant par le mélange DCM/AcOEt (90/10 ; v/v). On sépare les diastéréoisomères et recueille le composé le plus polaire. On obtient 0,8 g du produit attendu après cristallisation dans l'heptane, F = 155°C.
$\alpha_D^{20}$ = -221° (c = 0,23 ; chloroforme)
RMN $^1H$ : DMSO-$d_6$ : δ (ppm) : 2,0 à 2,6 : 2se : 8H ; 2,8 : se : 3H ; 3,0 à 5,0 : m : 9H ; 6,0 à 7,2 : m : 10H ; 7,4 : d : 1H ; 8,0 : mt ; 2H.

EXEMPLE 7

(3S)-2-[5-Chloro-1-[(2,4-diméthoxyphényl)sulfonyl]-3-(2-méthoxyphényl)-6-méthyl-2-oxo-2,3-dihydro-1*H*-indol-3-yl] -N,N-diméthyl-1,2,3,4-tétrahydro isoquinoléine-3-carboxamide, isomère lévogyre.

(Ia) : $R_1$ = Cl ; $R_2$ = 6-$CH_3$ ; $R_3$ = $OCH_3$ ; $R_4$ = H ; $R_5$ = $N(CH_3)_2$ ; $R_6$ = 2-$OCH_3$ ; $R_7$ = $OCH_3$ ; $R_8$ = H ; $R_9$ =

H ; n = 1 ;p = 1.

On prépare ce composé selon le mode opératoire décrit à l'Exemple 1 à partir de 1,4 g du composé obtenu à la Préparation 3.6 dans 15 ml de DMF, 0,123 g d'hydrure de sodium à 60 % dans l'huile et 0,7 g de chlorure de 2,4-diméthoxybenzènesulfonyle. Après chromatographie sur gel de silice en éluant par le mélange DCM/AcOEt (90/10 ; v/v), on sépare les diastéréoisomères et recueille le composé le plus polaire. On obtient 0,88 g du produit attendu après cristallisation dans l'hexane, F = 160°C.

$\alpha_D^{20}$ = -227° (c = 0,18 ; chloroforme)

RMN $^1$H : DMSO-$d_6$ : δ (ppm) : 2,2 à 2,8 : m : 11H ; 3,6 : s : 3H ; 3,8 : d : 2H ; 4,4 : se : 1H ; 6,5 à 7,4: mt : 9H ; 7,6 : dd : 1H ; 10,2 : 2s : 1H.

En procédant selon les méthodes opératoires décrits dans les Exemples ci-dessus, à partir des composés de formule (II) décrits dans les Préparations 3 et le chlorure de 2,4-diméthoxybenzènesulfonyle, on prépare les composés selon l'invention rassemblés dans le TABLEAU I ci-après :

TABLEAU I

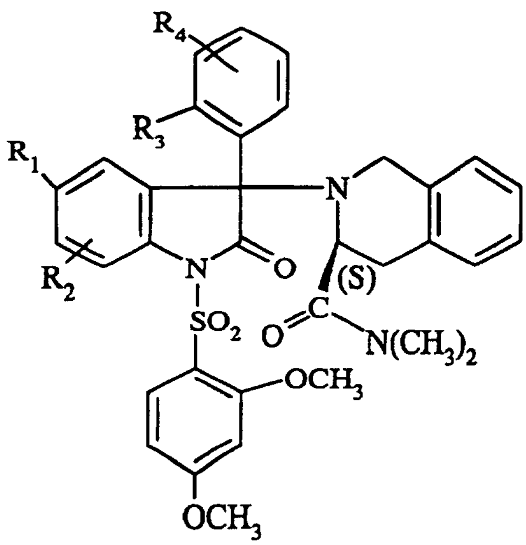

(Ia) : $R_8$ = H ; $R_9$ = H ;
n = 1 ; p = 1.

| Exemples | $R_1$ | $R_2$ | $R_3$ | $R_4$ | F°C ; solvant de cristallisation ; $\alpha_D^{20}$ (chloroforme) |
|---|---|---|---|---|---|
| 8 (a) | Cl | H | Cl | H | 155 heptane -254° (c = 0,18) |
| 9 (b) | Cl | H | $OCH_3$ | 3-$OCH_3$ | 215 éther iso -275° (c = 0,14) |
| 10 (c) | Cl | H | $OCH_3$ | 4-$OCH_3$ | 145 heptane/éther -252° (c = 0,3) |
| 11 (d) | Cl | H | 2,3-O-$CH_2$-O- | | 160 hexane -233° (c = 0,25) |

| | | | | | |
|---|---|---|---|---|---|
| 12 (e) | Cl | 6-Cl | Cl | H | 234 hexane/éther iso -334° (c = 0,22) |
| 13 (f) | Cl | 6-$OCH_3$ | Cl | H | 178 heptane -323° (c = 0,19) |
| 14 (g) | $CH_3$ | 4-Cl | $OCH_3$ | H | 146 éther iso -282° (c = 0,28) |

(a) Composé préparé selon le mode opératoire décrit à l'Exemple 1 à partir du composé obtenu à la Préparation 3.7. On chromatographie sur gel de silice en éluant par le mélange DCM/AcOEt (94/6 ; v/v) et recueille le composé le plus polaire.

(b) Composé préparé selon le mode opératoire décrit à l'Exemple 1 à partir du composé obtenu à la Préparation 3.8 On chromatographie sur gel de silice en éluant par le mélange DCM/AcOEt (90/10 ; v/v) et recueille le composé le plus polaire.

(c) Composé préparé selon le mode opératoire décrit à l'Exemple 1 à partir du composé obtenu à la Préparation 3.9 On chromatographie sur gel de silice en éluant par le mélange DCM/AcOEt (85/15 ; v/v) et recueille le composé le plus polaire.

(d) Composé préparé selon le mode opératoire décrit à l'Exemple 1 à partir du composé obtenu à la Préparation 3.10 On chromatographie sur gel de silice en éluant par le mélange DCM/AcOEt (93/7 ; v/v) et recueille le composé le plus polaire.

(e) Composé préparé selon le mode opératoire décrit à l'Exemple 1 à partir du composé obtenu à la Préparation 3.11 On chromatographie sur gel de silice en éluant par le mélange DCM/AcOEt (90/10 ; v/v) et recueille le composé le plus polaire.

(f) Composé préparé selon le mode opératoire décrit à l'Exemple 1 à partir du composé obtenu à la Préparation 3.12 On chromatographie sur gel de silice en éluant par le mélange DCM/AcOEt (90/10 ; v/v) et recueille le composé le plus polaire.

(g) Composé préparé selon le mode opératoire décrit à l'Exemple 1 à partir du composé obtenu à la Préparation 3.13 On chromatographie sur gel de silice en éluant par le mélange DCM/AcOEt (85/15 ; v/v) et recueille le composé le plus polaire.

## Revendications

**1.** Composé de formule (Ia) sous forme d'isomère lévogyre :

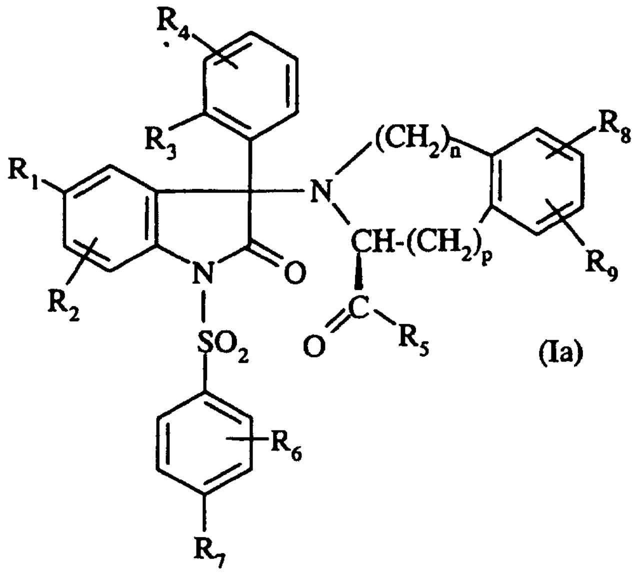

(Ia)

dans laquelle :
l'atome de carbone portant le substituant $COR_5$ a la configuration (S),

- le radical

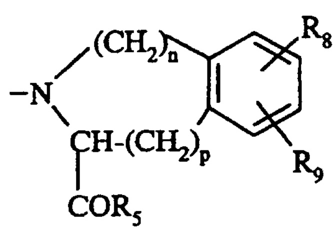

représente un radical :

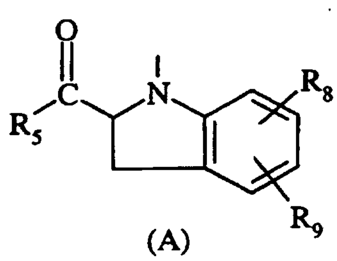

(A) ;

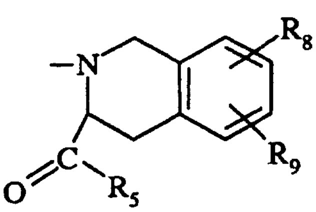

(D) ;

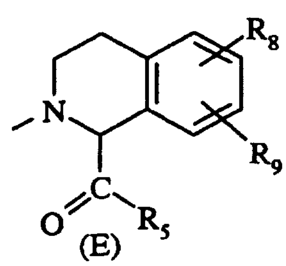

(E)

- $R_1$ représente un atome de chlore ou un radical méthyle ;
- $R_2$ représente un atome d'hydrogène ou est en position -4- ou -6- de l'indol-2-one et représente un atome de chlore, un radical méthyle ou un radical méthoxy ;
- $R_3$ représente un radical méthoxy ou un atome de chlore ;
- $R_4$ représente un atome d'hydrogène ou est en position -3- ou -4- du phényle et représente un radical méthoxy ;
- ou bien $R_4$ est en position -3- du phényle et ensemble avec $R_3$ représentent un radical méthylènedioxy ;

- $R_5$ représente un groupe diméthylamino ou un radical méthoxy ;
- $R_6$ est en position -2- du phényle et représente un radical méthoxy ;
- $R_7$ représente un radical méthoxy ;
- $R_8$ et $R_9$ représentent un atome d'hydrogène ;

ainsi que leurs solvats et/ou hydrates.

**2.** Un composé selon la revendication 1 choisi parmi :

- (3S)-2-[5-Chloro-1-[(2,4-diméthoxyphényl)sulfonyl]-3-(2-méthoxyphényl)-2-oxo-2,3-dihydro-1*H*-indol-3-yl]-N, N-diméthyl-1,2,3,4-tétrahydroisoquinoléine-3-carboxamide, isomère lévogyre ;
- (1S)-2-[5-Chloro-1-[(2,4-diméthoxyphényl)sulfonyl]-3-(2-méthoxyphényl)-2-oxo-2,3-dihydro-1*H*-indol-3-yl]-N, N-diméthyl-1,2,3,4-tétrahydroisoquinoléine-1-carboxamide, isomère lévogyre ;
- (2S)-1-[5-Chloro-1-[(2,4-diméthoxyphényl)sulfonyl]-3-(2-méthoxyphényl)-2-oxo-2,3-dihydro-1*H*-indol-3-yl]-N, N-diméthyl-2,3-dihydro-1H-indole-2-carboxamide, isomère lévogyre ;
- Ester méthylique de l'acide (3S)-2-[5-chloro-1-[(2,4-diméthoxyphényl)sulfonyl]-3-(2-méthoxyphényl)-2-oxo-2,3-dihydro-1*H*-indol-3-yl]-1,2,3,4-tétrahydroiso quinoléine-3-carboxylique, isomère lévogyre ;
- (3S)-2-[5,6-Dichloro-1-[(2,4-diméthoxyphényl)sulfonyl]-3-(2-méthoxyphényl)-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-1-[(2,4-diméthoxyphényl)sulfonyl]-3-(2-méthoxyphényl)-6-méthyl-2-oxo-2,3-dihydro-1*H*-indol-3-yl]-N,N-diméthyl-1,2,3,4-tétrahydro isoquinoléine-3-carboxamide, isomère lévogyre ;
- (3S)-2-[5-Chloro-3-(2-chlorophényl)-1-[(2,4-diméthoxyphényl)sulfonyl]-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-1-[(2,4-diméthoxyphényl)sulfonyl]-3-(2,3-diméthoxyphényl)-2-oxo-2,3-dihydro-1H-indol-3-yl]-N,N-diméthyl-1,2,3,4-tétrahydroisoquinoléine-3-carboxamide, isomère lévogyre ;
- (3S)-2-[5-Chloro-1-[(2,4-diméthoxyphényl)sulfonyl]-3-(2,4-diméthoxyphényl)-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5-Chloro-3-(1,3-benzodioxol-4-yl)-1-[(2,4-diméthoxyphényl)sulfonyl]-2-oxo-2,3-dihydro-1*H*-indol-3-yl]-N,N-diméthyl-1,2,3,4-tétrahydroisoquinoléine-3-carboxamide, isomère lévogyre ;
- (3S)-2-[5,6-Dichloro-3-(2-chlorophényl)-1-[(2,4-diméthoxyphényl)sulfonyl]-2-oxo-2,3-dihydro-1*H*-indol-3-yl] -N,N-diméthyl-1,2,3,4-tétrahydroisoquinoléine-3-carboxamide, isomère lévogyre ;
- (3S)-2-[5-Chloro-3-(2-chlorophényl)-1-[(2,4-diméthoxyphényl)sulfonyl]-6-méthoxy-2-oxo-2,3-dihydro-1*H*-indol-3-yl]-N,N-diméthyl-1,2,3,4-tétrahydroiso quinoléine-3-carboxamide, isomère lévogyre ;
- (3S)-2-[4-Chloro-1-[(2,4-diméthoxyphényl)sulfonyl]-3-(2-méthoxyphényl)-5-méthyl-2-oxo-2,3-dihydro-1*H*-indol-3-yl]-N,N-diméthyl-1,2,3,4-tétrahydroiso quinoléine-3-carboxamide, isomère lévogyre ;

ainsi que ses solvats et/ou hydrates.

**3.** Procédé de préparation des composés de formule (Ia) isomère lévogyre selon la revendication 1, de leurs solvats et/ou leurs hydrates **caractérisé en ce que** : on fait réagir, en présence d'une base, un composé de formule :

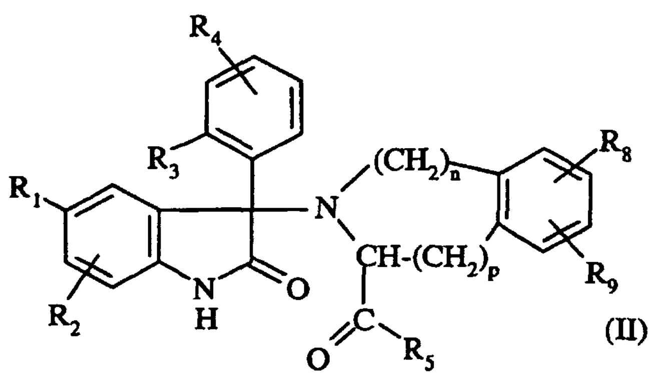

(II)

dans laquelle l'atome de carbone portant le substituant $COR_5$ a la configuration (S), n, p, $R_1$, $R_2$, $R_3$, $R_4$, $R_5$, $R_8$ et $R_9$ sont tels que définis pour un composé de formule (Ia) isomère lévogyre dans la revendication 1, avec un halogénure de formule :

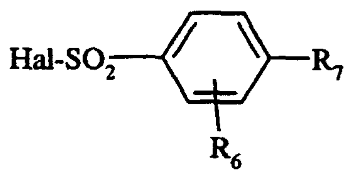

(III)

dans laquelle $R_6$ et $R_7$ sont tels que définis pour un composé de formule isomère lévogyre dans la revendication 1 et Hal représente un atome d'halogène.

**4.** Composé de formule :

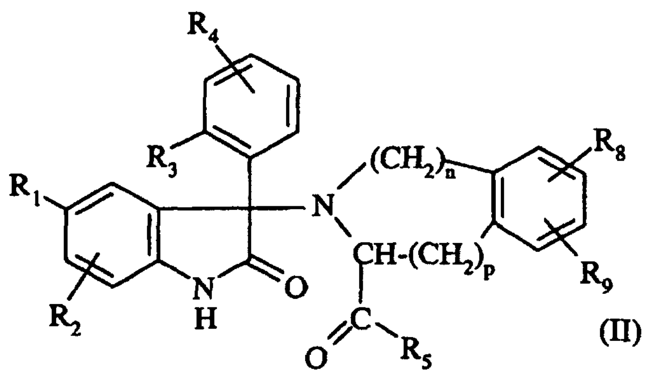

(II)

dans laquelle :

- l'atome de carbone portant le substituant $COR_5$ a la configuration (S) ;
- le radical

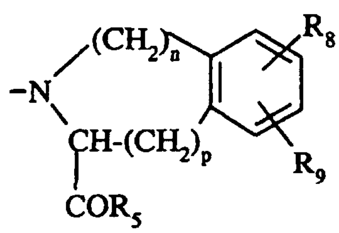

,

$R_1$, $R_2$, $R_3$, $R_4$, $R_5$, $R_8$ et $R_9$ sont tels que définis pour un composé de formule (Ia) isomère lévogyre dans la revendication 1 ;

ainsi que leurs sels avec des acides minéraux ou organiques, sous forme d'isomères optiquement purs ou sous forme de mélange de diastéréoisomères ou sous forme de mélange racémique.

**5.** Composition pharmaceutique comprenant en tant que principe actif un composé selon la revendication 1 ou 2, un de ses solvats et/ou hydrates pharmaceutiquement acceptables.

**6.** Utilisation d'un composé selon la revendication 1 ou 2, de ses solvats et/ou hydrates pharmaceutiquement acceptables, pour la préparation de médicaments destinés à traiter toute pathologie où l'arginine-vasopressine et/ou ses récepteurs $V_{1b}$ ou la fois ses récepteurs $V_{1b}$ et ses récepteurs $V_{1a}$ sont impliqués.

**7.** Médicament **caractérisé en ce qu'**il est constitué d'un composé selon la revendication 1 ou 2.

**Patentansprüche**

1. Verbindung der Formel (Ia) in Form des linksdrehenden Isomeren:

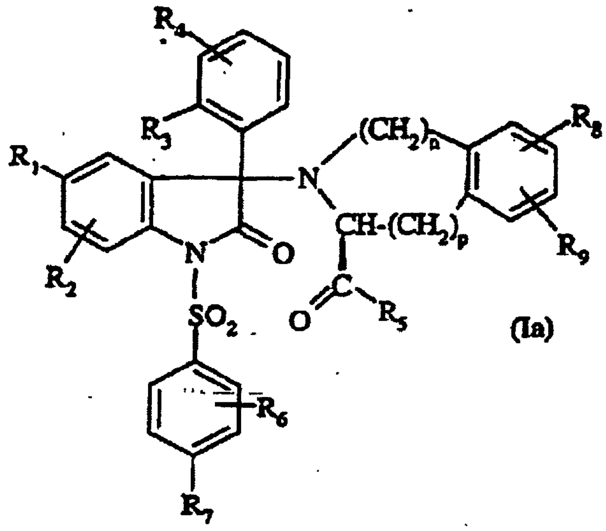

(Ia)

in der:
das den Substituenten $COR_5$ tragende Kohlenstoffatom die Konfiguration (S) aufweist,

- die Gruppe

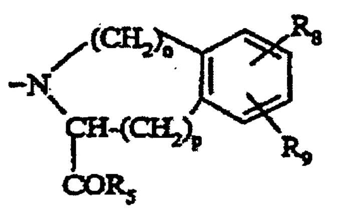

eine Gruppe:

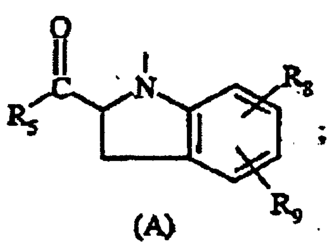

(A) ;

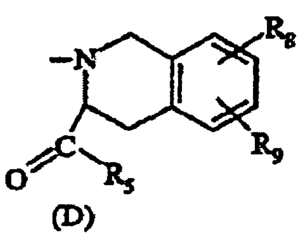

(D) ;

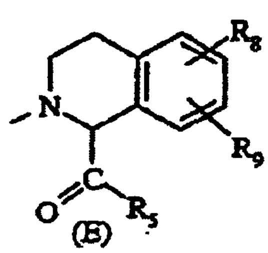

(E)

darstellt;
- $R_1$ ein Chloratom oder eine Methylgruppe bedeutet;
- $R_2$ ein Wasserstoffatom bedeutet oder in der Position -4- oder -6- des Indol-2-ons steht und ein Chloratom, eine Methylgruppe oder eine Methoxygruppe darstellt;
- $R_3$ eine Methoxygruppe oder ein Chloratom bedeutet;

- $R_4$ ein Wasserstoffatom bedeutet oder in der Stellung -3- oder -4- des Phenyls steht und eine Methoxygruppe bedeutet:

- oder $R_4$ in der Stellung -3- des Phenyls steht und gemeinsam mit $R_3$ eine Methylendioxygruppe bedeutet;
- $R_5$ eine Dimethylaminogruppe oder eine Methoxygruppe bedeutet;
- $R_6$ in der Stellung -2- des Phenyls steht und eine Methoxygruppe bedeutet;
- $R_7$ eine Methoxygruppe bedeutet;
- $R_8$ und $R_9$ ein Wasserstoffatom bedeuten;

sowie deren Solvate und/oder Hydrate.

**2.** Verbindung nach Anspruch 1, ausgewählt aus:

- (3S)-2-[5-Chlor-1-[(2,4-dimethoxyphenyl)-sulfonyl]-3-(2-methoxyphenyl)-2-oxo-2,3-dihydro-1*H*-indol-3-yl]-N,N-dimethyl-1,2,3,4-tetrahydroisochinolin-3-carboxamid, linksdrehendes Isomeres;
- (1S)-2-[5-Chlor-1-[(2,4-dimethoxyphenyl)-sulfonyl]-3-(2-methoxyphenyl)-2-oxo-2,3-dihydro-1*H*-indol-3-yl]-N,N-dimethyl-1,2,3,4-tetrahydroisochinolin-1-carboxamid, linksdrehendes Isomeres;
- (2S)-1-[5-Chlor-1-[(2,3-dimethoxyphenyl)-sulfonyl]-3-(2-methoxyphenyl)-2-oxo-2,3-dihydro-1*H*-indol-3-yl]-N,N-dimethyl-2,3-1H-indol-2-carboxamid, linksdrehendes Isomeres;
- (3S)-2-[5-Chlor-1-[(2,4-dimethoxyphenyl)-sulfonyl]-3-(2-methoxyphenyl)-2-oxo-2,3-dihydro-1*H*-indol-3-yl]-1,2,3,4-tetrahydroisochinolin-3-carbonsäuremethylester, linksdrehendes Isomeres;
- (3S)-2-[5,6-Dichlor-1-[(2,4-dimethoxyphenyl)-sulfonyl]-3-(2-methoxyphenyl)-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5-Chlor-1-[(2,4-dimethoxyphenyl)-sulfonyl]-3-(2-methoxyphenyl)-6-methyl-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5-Chlor-3-(2-chlorphenyl)-1-[(2,4-dimethoxyphenyl)-sulfonyl]-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5-Chlor-1-[(2,4-dimethoxyphenyl)-sulfonyl]-3-(2,3-dimethoxyphenyl)-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5-Chlor-1-[(2,4-dimethoxyphenyl)-sulfonyl]-3-(2,4-dimethoxyphenyl)-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5-Chlor-3-(1,3-benzodioxol-5-yl)-1-[(2,4-dimethoxyphenyl)-sulfonyl]-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5,6-Dichlor-3-(2-chlorphenyl)-1-[(2,4-dimethoxyphenyl)-sulfonyl]-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[5-Chlor-3-(2-chlorphenyl)-1-[(2,4-dimethoxyphenyl)-sulfonyl]-6-methoxy-2-oxo-2,3-dihydro-1*H*-indol-3-yl]-N,N-dimethyl-1,2,3,4-tetrahydroisochinolin-3-carboxamid, linksdrehendes Isomeres;
- (3S)-2-[4-Chlor-1-[(2,4-dimethoxyphenyl)-sulfonyl]-3-(2-methoxyphenyl)-5-meethyl-2-oxo-2,3-dihydro-1*H*-indol-3-yl]-N,N-dimethyl-1,2,3,4-tetrahydroisochinolin-3-carboxamid, linksdrehendes Isomeres;

sowie deren Solvate und/oder Hydrate.

**3.** Verfahren zur Herstellung der Verbindungen der Formel (Ia) in Form der linksdrehenden Isomeren nach Anspruch 1, von deren Solvaten und/oder deren Hydraten, **dadurch gekennzeichnet, daß** man eine Verbindung der Formel:

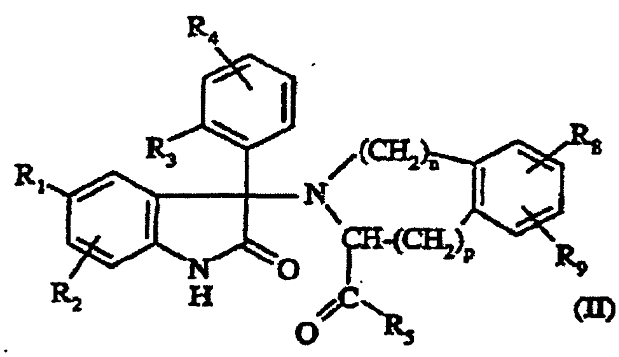

(II)

in der das den Substituenten $COR_5$ tragende Kohlenstoffatom die Konfiguration (S) aufweist und n, p, $R_1$, $R_2$, $R_3$, $R_4$, $R_5$, $R_8$ und $R_9$ die für die Verbindung der Formel (Ia) in Form des linksdrehenden Isomeren die in Anspruch 1 angegebenen Bedeutungen besitzen, in Gegenwart einer Base mit einem Halogenid der Formel umsetzt:

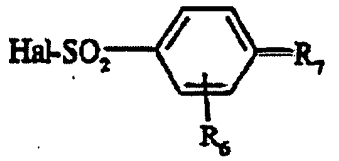

(III)

in der $R_6$ und $R_7$ die für die Verbindung der Formel (Ia) in Form des linksdrehenden Isomeren in Anspruch 1 angegebenen Bedeutungen besitzt und Hal einHalogenatom darstellt.

**4.** Verbindung der Formel:

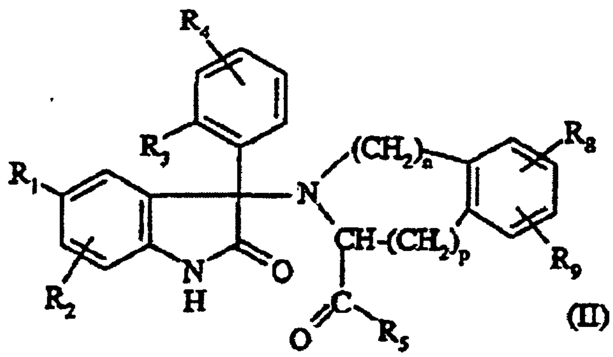

(II)

in der:

- das den Substituenten $COR_5$ tragende Kohlenstoffatom die Konfiguration (S) aufweist.
- die Gruppe

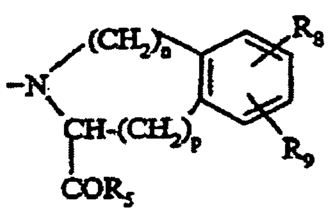

und die Gruppe $R_1$, $R_2$, $R_3$, $R_4$, $R_5$, $R_8$ und $R_9$ die bezüglich der Verbindung der Formel (Ia) in Form des linksdrehenden Isomeren die in Anspruch 1 angegebenen Bedeutungen besitzen;

sowie deren Salze mit anorganischen oder organischen Säuren, in Form der optisch reinen Isomeren oder in Form einer Mischung der Diastereoisomeren oder in Form einer racemischen Mischung.

**5.** Pharmazeutische Zubereitung enthaltend als Wirkstoff eine Verbindung nach Anspruch 1 oder 2 oder eines ihrer pharmazeutisch annehmbaren Solvate und/oder Hydrate.

**6.** Verwendung einer Verbindung nach Anspruch 1 oder 2 und ihren pharmazeutisch annehmbaren Solvaten und/oder Hydraten für die Herstellung von Arzneimitteln zur Behandlung von sämtlichen Erkrankungen, bei denen Arginin-Vasopressin und/oder seine Rezeptoren $V_{1b}$ oder gleichzeitig seine Rezeptoren $V_{1b}$ und seine Rezeptoren $V_{1a}$ beteiligt sind.

**7.** Arzneimittel, **dadurch gekennzeichnet, daß** es aus einer Verbindung nach Anspruch 1 oder 2 gebildet ist.

## Claims

**1.** Compound of formula (Ia) in the form of the levorotatory isomer:

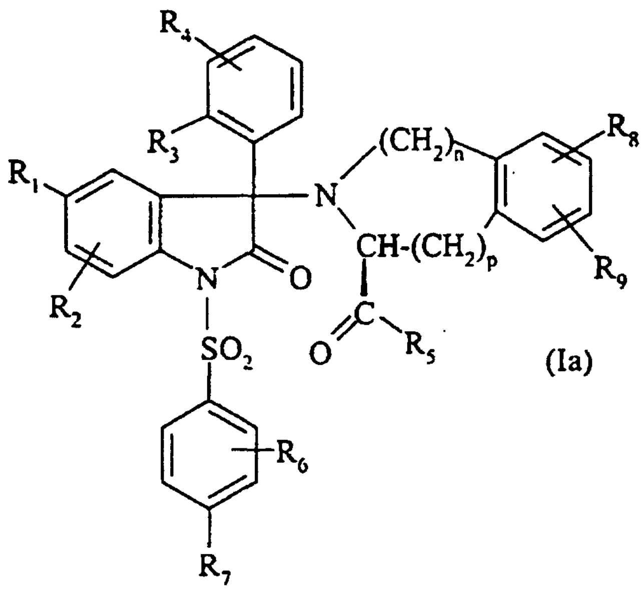

(Ia)

in which:
the carbon atom carrying the $COR_5$ substituent has the (S) configuration;

- the

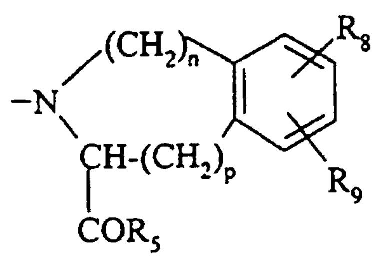

radical represents a radical:

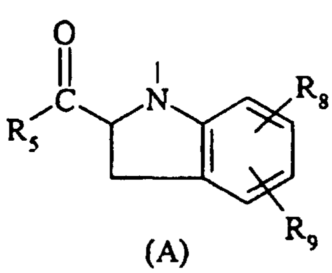

(A) ;

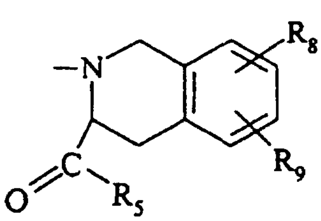

(D) ;

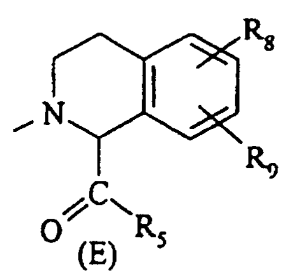

(E)

- $R_1$ represents a chlorine atom or a methyl radical;
- $R_2$ represents a hydrogen atom or is in the 4- or 6-position of the indol-2-one and represents a chlorine atom, a methyl radical or a methoxy radical;
- $R_3$ represents a methoxy radical or a chlorine atom;
- $R_4$ represents a hydrogen atom or is in the 3- or 4-position of the phenyl and represents a methoxy radical;
- or else $R_4$ is in the 3-position of the phenyl and together with $R_3$ represents a methylenedioxy radical;
- $R_5$ represents a dimethylamino group or a methoxy radical;
- $R_6$ is in the 2-position of the phenyl and represents a methoxy radical;
- $R_7$ represents a methoxy radical;
- $R_8$ and $R_9$ represent a hydrogen atom;

and its solvates and/or hydrates.

**2.** Compound according to Claim 1 chosen from:

- (3S)-2-[5-Chloro-1-[(2,4-dimethoxyphenyl)-sulphonyl]-3-(2-methoxyphenyl)-2-oxo-2,3-dihydro-1*H*-indol-3-yl]-N,N-dimethyl-1,2,3,4-tetrahydroisoquinoline-3-carboxamide, levorotatory isomer;
- (1S)-2-[5-Chloro-1-[(2,4-dimethoxyphenyl)-sulphonyl]-3-(2-methoxyphenyl)-2-oxo-2,3-dihydro-1*H*-indol-3-yl]-N,N-dimethyl-1,2,3,4-tetrahydroisoquinoline-1-carboxamide, levorotatory isomer;
- (2S)-1-[5-Chloro-1-[(2,4-dimethoxyphenyl)-sulphonyl]-3-(2-methoxyphenyl)-2-oxo-2,3-dihydro-1*H*-indol-3-yl]-N,N-dimethyl-2,3-dihydro-1H-indole-2-carboxamide, levorotatory isomer;
- Methyl ester of (3S)-2-[5-chloro-1-[(2,4-dimethoxyphenyl)sulphonyl]-3-(2-methoxyphenyl)-2-oxo-2,3-dihydro-1*H*-indol-3-yl]-1,2,3,4-tetrahydroisoquinoline-3-carboxylic acid, levorotatory isomer;
- (3S)-2-[5,6-Dichloro-1-[(2,4-dimethoxyphenyl)-sulphonyl]-3-(2-methoxyphenyl)-2-oxo-2,3-dihydro-1*H*-indol-3-yl]-N,N-dimethyl-1,2,3,4-tetrahydroisoquinoline-3-carboxamide, levorotatory isomer;
- (3S)-2-[5-Chloro-1-[(2,4-dimethoxyphenyl)sulphonyl]-3-(2-methoxyphenyl)-6-methyl-2-oxo-2,3-dihydro-1*H*-indol-3-yl]-N,N-dimethyl-1,2,3,4-tetrahydroisoquinoline-3-carboxamide, levorotatory isomer;
- (3S)-2-[5-Chloro-3-(2-chlorophenyl)-1-[(2,4-dimethoxyphenyl)sulphonyl]-2-oxo-2,3-dihydro-1*H*-indol-3-yl]-N,N-dimethyl-1,2,3,4-tetrahydroisoquinoline-3-carboxamide, levorotatory isomer;
- (3S)-2-[5-Chloro-1-[(2,4-dimethoxyphenyl)sulphonyl]-3-(2,3-dimethoxyphenyl)-2-oxo-2,3-dihydro-1*H*-indol-3-yl]-N,N-dimethyl-1,2,3,4-tetrahydroisoquinoline-3-carboxamide, levorotatory isomer;
- (3S)-2-[5-Chloro-1-[(2,4-dimethoxyphenyl)sulphonyl]-3-(2,4-dimethoxyphenyl)-2-oxo-2,3-dihydro-1*H*-indol-3-yl]-N,N-dimethyl-1,2,3,4-tetrahydroisoquinoline-3-carboxamide, levorotatory isomer;
- (3S)-2-[5-Chloro-3-(1,3-benzodioxol-4-yl)-1-[(2,4-dimethoxyphenyl)sulphonyl]-2-oxo-2,3-dihydro-1*H*-indol-3-yl]-N,N-dimethyl-1,2,3,4-tetrahydroisoquinoline-3-carboxamide, levorotatory isomer;
- (3S)-2-[5,6-Dichloro-3-(2-chlorophenyl)-1-[(2,4-dimethoxyphenyl)sulphonyl]-2-oxo-2,3-dihydro-1*H*-indol-3-yl]-N,N-dimethyl-1,2,3,4-tetrahydroisoquinoline-3-carboxamide, levorotatory isomer;
- (3S)-2-[5-Chloro-3-(2-chlorophenyl)-1-[(2,4-dimethoxyphenyl)sulphonyl]-6-methoxy-2-oxo-2,3-dihydro-1*H*-indol-3-yl]-N,N-dimethyl-1,2,3,4-tetrahydroisoquinoline-3-carboxamide, levorotatory isomer;
- (3S)-2-[4-Chloro-1-[(2,4-dimethoxyphenyl)sulphonyl]-3-(2-methoxyphenyl)-5-methyl-2-oxo-2,3-dihydro-1*H*-indol-3-yl]-N,N-dimethyl-1,2,3,4-tetrahydroisoquinoline-3-carboxamide, levorotatory isomer;

and its solvates and/or hydrates.

**3.** Process for the preparation of the compounds of formula (Ia), levorotatory isomer, according to Claim 1, their solvates and/or their hydrates, **characterized in that**:
a compound of formula:

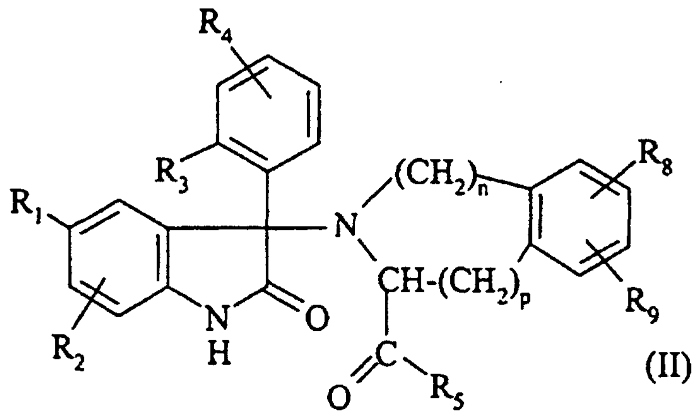

(II)

in which the carbon atom carrying the $COR_5$ substituent has the (S) configuration and n, p, $R_1$, $R_2$, $R_3$, $R_4$, $R_5$, $R_8$ and $R_9$ are as defined for a compound of formula (Ia), levorotatory isomer, in Claim 1, is reacted, in the presence of a base, with a halide of formula:

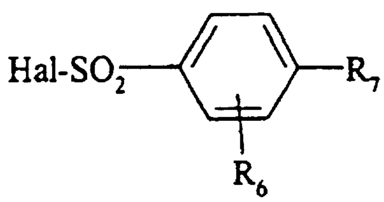

(III)

in which $R_6$ and $R_7$ are as defined for a compound of formula (Ia), levorotatory isomer, in Claim 1 and Hal represents a halogen atom.

**4.** Compound of formula:

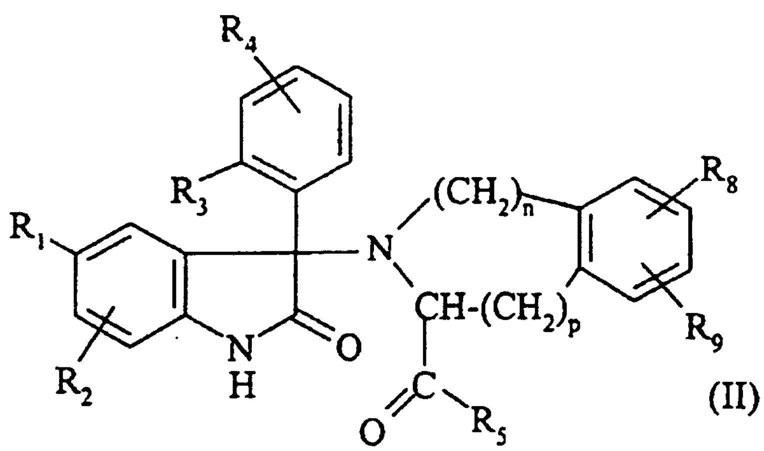

(II)

in which:

- the carbon atom carrying the $COR_5$ substituent has the (S) configuration;
- the

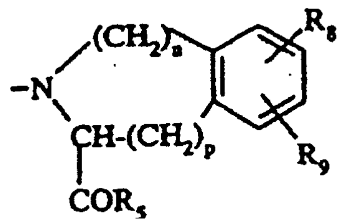

radical and $R_1$, $R_2$, $R_3$, $R_4$, $R_5$, $R_8$ and $R_9$ are as defined for a compound of formula (Ia), levorotatory isomer, in Claim 1;

and its salts with inorganic or organic acids, in the form of optically pure isomers or in the form of a mixture of diastereoisomers or in the form of a racemic mixture.

**5.** Pharmaceutical composition comprising, as active principle, a compound according to Claim 1 or 2, one of its solvates and/or one of its hydrates which are pharmaceutically acceptable.

**6.** Use of a compound according to Claim 1 or 2, of its solvates and/or of its hydrates which are pharmaceutically acceptable, for the preparation of medicaments intended for the treatment of any pathology where arginine-vasopressin and/or its $V_{1b}$ receptors or both its $V_{1b}$ receptors and its $V_{1a}$ receptors are implicated.

**7.** Medicament, **characterized in that** it is composed of a compound according to Claim 1 or 2.